# EUROPEAN PATENT APPLICATION

(11) **EP 4 293 021 A1**
(43) Date of publication of application: **20.12.2023**
(21) Application number: 22752277.8
(22) Date of filing: 09.02.2022
(51) Int. Cl.: C07D 471/04, A61K 31/506, A61P 35/00

(54) **SALT OF NITROGEN-CONTAINING FUSED HETEROCYCLIC COMPOUND OR CRYSTAL FORM THEREOF, AND PREPARATION METHOD THEREFOR, PHARMACEUTICAL COMPOSITION THEREOF, AND USE THEREOF**

(30) Priority: 10.02.2021 CN 202110185149
(71) Applicant: Shanghai Pharmaceuticals Holding Co., Ltd., Shanghai 201203 (CN)
(72) Inventor: CHEN, Hui, Shanghai 201203 (CN); XIA, Guangxin, Shanghai 201203 (CN); WANG, Qian, Shanghai 201203 (CN); LIU, Junyao, Shanghai 201203 (CN); HAN, Yanan, Shanghai 201203 (CN); KE, Ying, Shanghai 201203 (CN)
(74) Representative: Petraz, Gilberto Luigi
(86) International application number: PCT/CN2022/075631
(87) International publication number: WO 2022/171117

(57) **Abstract**

A salt of a nitrogen-containing fused heterocyclic compound or a crystal form thereof, and a preparation method therefor, a pharmaceutical composition thereof, and a use thereof, in particular, a salt of compound I as shown below or a crystal form thereof, and a preparation method, a pharmaceutical composition and a use. The salt of compound I or the crystal form thereof exhibits at least one of the following advantages: improved bioavailability, great mechanical properties, improved chemical stability, excellent fluidity, great compressibility, and improved dissolution characteristics.

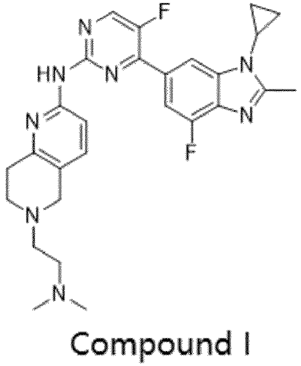

## Description

### Technical field

The present invention relates to N-(4-(1-cyclopropyl-4-fluoro-2-methyl-1H-benzimidazol-6-yl)-5-fluoropyrimidin-2-yl)-6-(2-(dimethylamino)ethyl)-5,6,7,8-tetrahydro-1,6-naphthyridin-2-amine, specifically, to a salt of the compound, a crystal form, a preparation method and a pharmaceutical composition and use thereof.

### Background

Compound I, N-(4-(1-cyclopropyl-4-fluoro-2-methyl-1H-benzimidazol-6-yl)-5-fluoropyrimidin-2-yl)-6-(2-(dimethylamino)ethyl)-5,6,7,8-tetrahydro-1,6-naphthyridin-2-amine, has a structure of the following formula:

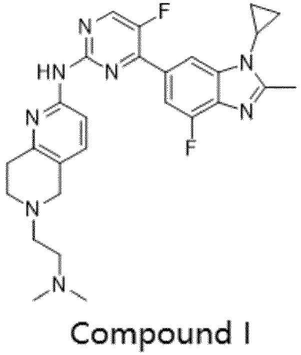

Compound I has been disclosed in CN106928219A and WO2017114512A1, the entire disclosures of which are hereby incorporated by reference as if they are described herein. It has high selectivity and high inhibitory activity at the molecular level to cyclin-dependent kinase 4 (CDK4) and cyclin-dependent kinases 6 (CDK6), and has a significant inhibitory effect on tumor cells associated with cyclin-dependent kinase activity at the cellular level and animal level, and can be used for the treatment of malignant tumors such as breast cancer, colon cancer, non-small cell carcinoma, brain astrocytoma, chronic myelogenous leukemia, pancreatic cancer, acute monocytic leukemia, liver cancer (including hepatocellular carcinoma, hepatic adenocarcinoma), gastric cancer, non-small cell lung cancer, malignant glioblastoma and prostate adenocarcinoma, and it has good liver microsome stability in humans, mice, etc. without obvious inhibition on metabolic enzymes, and it has good in vivo absorption properties in rats and mice and high bioavailability with good druggability.

However, there is still a need to provide Compound I with desirable processing properties such as ease of handling, ease of processing, improved dissolution profile, improved shelf life, ease of purification etc. to improve the performance characteristics of the drug.

### Summary of the invention

The present invention provides salts of Compound I, crystal forms and preparation methods therefor, pharmaceutical compositions and uses thereof. The salts of Compound I or crystal forms thereof exhibit at least one of the following advantages: improved bioavailability, good mechanical properties, improved chemical stability, excellent flow properties, good compressibility and improved dissolution properties.

In one aspect, the present invention provides a salt of Compound I as shown below, wherein the salt is selected from the group consisting of maleate, hydrochloride, phosphate, lactate, fumarate, succinate, malate, adipate, hippurate, glycolate, benzoate and nicotinate.

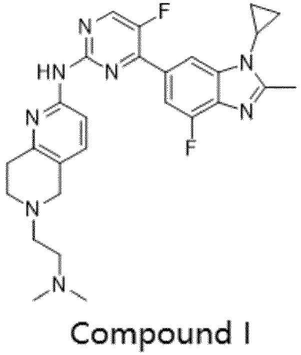

The salt of Compound I can be obtained by conventional methods for preparing a salt compound in the art, for example, the salt can be obtained by reacting the free base of Compound I with a corresponding acid, or can be obtained through an acid displacement reaction, or can be obtained according to the methods described below or in the examples in the present application, in combination with common technical knowledge in the art.

The salt can be one or more selected from the group consisting of maleate crystal form A, maleate crystal form B, maleate crystal form C, hydrochloride crystal form A, hydrochloride crystal form B, phosphate crystal form A, lactate crystal form A, fumarate crystal form A, succinate crystal form A, malate crystal form A, adipate crystal form A, hippurate crystal form A, glycolate crystal form A, benzoate crystal form A and nicotinate crystal form A of Compound I.

More particularly, the salt can be one or more selected from the group consisting of maleate crystal form A, fumarate crystal form A, adipate crystal form A and benzoate crystal form A of Compound I.

More particularly, the salt may be maleate crystal form A of Compound I.

More particularly, the salt may be fumarate crystal form A of Compound I.

More particularly, the salt may be adipate crystal form A of Compound I.

More particularly, the salt may be benzoate crystal form A of Compound I.

In one embodiment, the salt of Compound I is a maleate.

In one embodiment, the maleate of Compound I exists in the form of crystal form A, crystal form B or crystal form C.

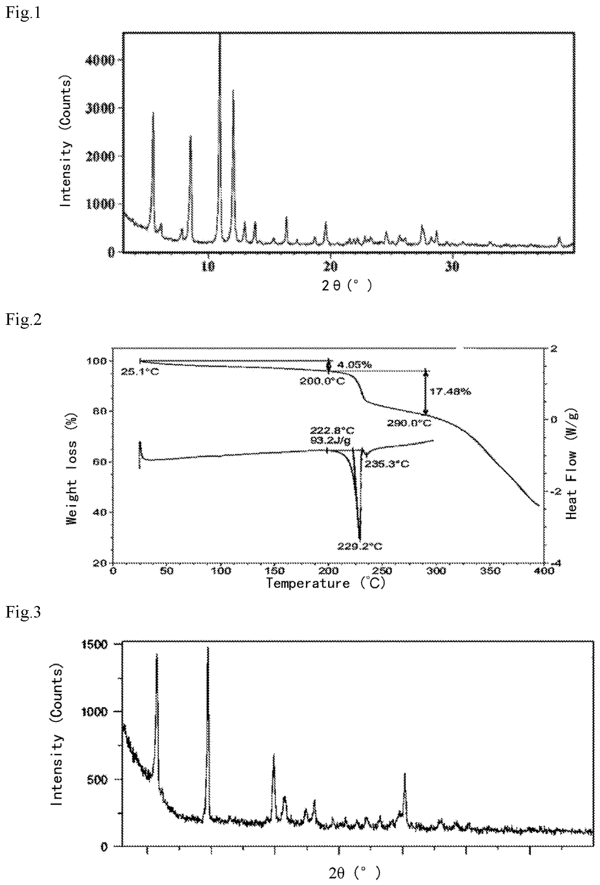

The X-ray powder diffraction (XRPD) pattern of the maleate crystal form A has characteristic peaks at diffraction angles 2θ of about 5.48±0.2°, 8.55±0.2°, 10.92±0.2°, 12.04±0.2°, 12.98±0.2°, 13.81±0.2°, 16.40±0.2°, 19.59±0.2°, and 27.46±0.2°, preferably has further diffraction peaks at one or more positions of 6.11±0.2°, 7.84±0.2°, 15.32±0.2°, 18.70±0.2°, 21.55±0.2°, 22.21±0.2°, 22.80±0.2°, 23.32±0.2°, 24.57±0.2°, 25.62±0.2°, 26.07±0.2°, 28.23±0.2°, 28.68±0.2°, 33.08±0.2°, and 38.76±0.2°, preferably has characteristic peaks at diffraction angles 2θ of about 5.48±0.2°, 6.11±0.2°, 7.84±0.2°, 8.55±0.2°, 10.92±0.2°, 12.04±0.2°, 12.98±0.2°, 13.81±0.2°, 15.32±0.2°, 16.40±0.2°, 18.70±0.2°, 19.59±0.2°, 21.55±0.2°, 22.21±0.2°, 22.80±0.2°, 23.32±0.2°, 24.57±0.2°, 25.62±0.2°, 26.07±0.2°, 27.46±0.2°, 28.23±0.2°, 28.68±0.2°, 33.08±0.2° and 38.76±0.2°, in particular, the maleate crystal form A has an XRPD pattern substantially as shown in FIG. 1, and the target type used in the XRPD is a Cu target.

The differential scanning calorimetry (DSC) graph of the maleate crystal form A has an endothermic peak with an onset temperature of about 222.8 °C and an endothermic peak with a peak temperature of about 235.3 °C, in particular, the maleate crystal form A has a DSC graph substantially as shown in FIG. 2.

The thermogravimetric (TGA) graph of the maleate crystal form A shows that the sample has a weight loss of about 4.1% at about 200 °C, and a total weight loss of about 17.5% in the range of about 200 to 290 °C, which is presumed to be caused by deacidification (the theoretical weight loss of deacidification is about 18.7%), in particular, the maleate crystal form A has a TGA graph substantially as shown in FIG. 2.

As detected by ¹H NMR, in the maleate crystal form A, the molar ratio of maleic acid to the free base is about 1:1. As detected by variable temperature XRPD (VT-XRPD), the maleate crystal form A does not change after being heated to about 170 °C and cooled to about 30 °C under the protection of N₂, thus it is speculated that the maleate crystal form A is an anhydrous crystal form.

The X-ray powder diffraction (XRPD) pattern of the maleate crystal form B has characteristic peaks at diffraction angles 2θ of about 5.75±0.2°, 9.72±0.2°, 14.91±0.2°, 15.76±0.2°, 17.43±0.2°, 18.09±0.2°, 22.20±0.2°, 23.23±0.2°, 25.17±0.2°, and 27.96±0.2°, in particular, the maleate crystal form B has an XRPD pattern substantially as shown in FIG. 3, and the target type used in the XRPD is a Cu target.

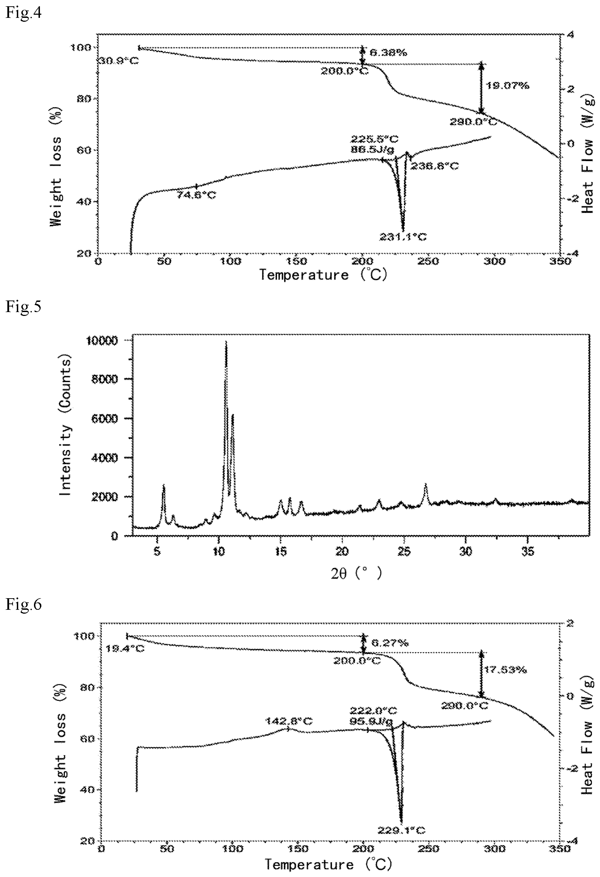

The differential scanning calorimetry (DSC) graph of the maleate crystal form B has two weak endothermic peaks with peak temperatures of about 74.6 °C and about 236.8 °C, respectively, and a sharp endothermic peak with an onset temperature of about 225.5 °C, in particular, the maleate crystal form B has a DSC graph substantially as shown in FIG. 4.

The thermogravimetric (TGA) graph of the maleate crystal form B shows that the sample has a weight loss of 6.4% when heated to about 200 °C and a total weight loss of about 19.1% in the range of about 200 to 290 °C, which is presumed to be caused by deacidification (the theoretical weight loss of the deacidification is about 18.7%), in particular, the maleate crystal form B has a TGA graph substantially as shown in FIG. 4.

As detected by ¹H NMR, in the maleate crystal form B, the molar ratio of maleic acid to the free base is about 1:1, and it contains a small amount of anti-solvent, such as acetone or toluene.

The X-ray powder diffraction (XRPD) pattern of the maleate crystal form C has characteristic peaks at diffraction angles 2θ of about 5.51±0.2°, 6.31±0.2°, 8.91±0.2°, 9.62±0.2°, 10.59±0.2°, 11.05±0.2°, 12.17±0.2°, 14.99±0.2°, 15.73±0.2°, 16.65±0.2°, 21.40±0.2°, 22.94±0.2°, 24.71±0.2°, 26.72±0.2°, 28.22±0.2°, 32.36±0.2° and 38.52±0.2°, in particular, the maleate crystal form C has an XRPD pattern substantially as shown in FIG. 5, and the target used in the XRPD is a Cu target.

The differential scanning calorimetry (DSC) graph of the maleate crystal form C has an exothermic peak with a peak temperature of about 142.8 °C, and a sharp endothermic peak with an onset temperature of about 222.0 °C, in particular, the maleate crystal form C has a DSC graph substantially as shown in FIG. 6.

The thermogravimetric (TGA) graph of the maleate crystal form C shows that the sample has a weight loss of 6.3% when heated to about 200 °C, and a total weight loss of about 17.5% in the range of about 200 to 290 °C, which is presumed to be caused by deacidification (the theoretical weight loss of the deacidification is about 18.7%), in particular, the maleate crystal form C has a TGA graph substantially as shown in FIG. 6.

As detected by ¹H NMR, in the maleate crystal form C, the molar ratio of maleic acid to the free base is about 1: 1, and no solvent remains. As detected by VT-XRPD, the maleate crystal form C does not change when heated to about 110 °C under the protection of N₂, thus it is speculated that the maleate crystal form C is an anhydrous crystal form.

The maleate crystal form B transforms into the maleate crystal form C after purged with N₂ at about 30 °C, and it is speculated that the maleate crystal form B is a hydrate. The maleate crystal forms B and C can also transform into the maleate crystal form A.

The present invention also provides a method for preparing the maleate crystal form A, which is one of the following methods:
Method I:
   (1) the maleate crystal form A is obtained by reacting the free base of Compound I with maleic acid in a solvent selected from ketone solvents (such as acetone), ether solvents (such as tetrahydrofuran (THF)), alcohol solvents (such as methanol (MeOH)) and ester solvents (such as ethyl acetate (EtOA));
Method II:
   (2) the maleate crystal form A is obtained by the transformation of maleate crystal form B or C.

Both the maleate crystal forms B and C are thermodynamically unstable crystals, and will eventually transform into the maleate crystal form A. Therefore, there is no particular limitation on the method for transforming the maleate crystal form B or C into the maleate crystal form A. For example, the maleate crystal form B or C can be transformed into the maleate crystal form A by suspending and stirring in a solvent selected from ketone solvents (such as acetone), ether solvents (such as tetrahydrofuran (THF)) and ester solvents (such as ethyl acetate (EtOA)), but the present invention is not limited thereto.

The present invention also provides a method for preparing the maleate crystal form B, comprising: adding an anti-solvent (such as acetone and toluene) dropwise to a solution of maleate of Compound I in a mixed solvent of methanol (MeOH)/dichloromethane (DCM) (1:1, v/v) for recrystallization, and placing the obtained solid under ambient humidity at room temperature.

The present invention also provides a method for preparing the maleate crystal form C, comprising: purging the maleate crystal form B with an inert gas (such as N₂).

In one embodiment, the salt of Compound I is a hydrochloride.

In one embodiment, the hydrochloride of Compound I exists in the form of crystal form A or crystal form B.

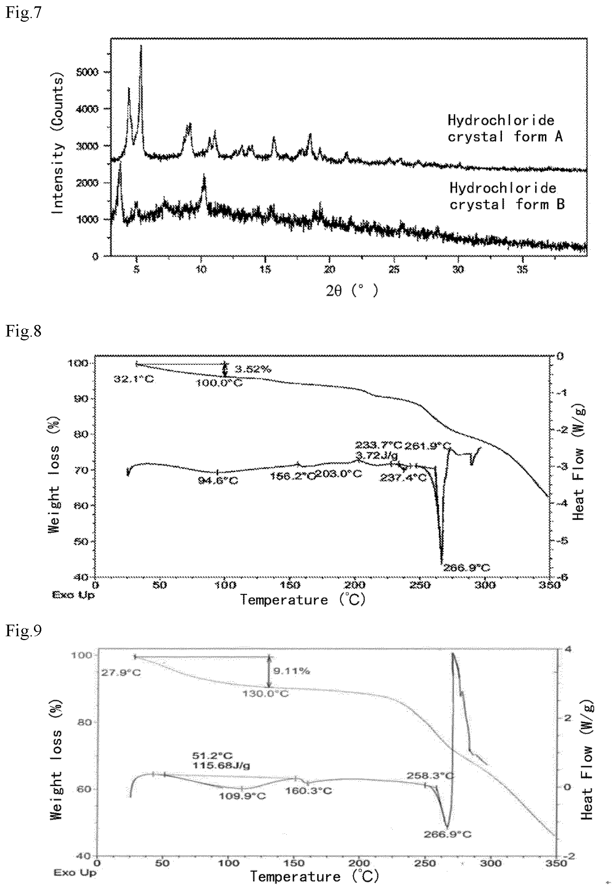

The X-ray powder diffraction (XRPD) pattern of the hydrochloride crystal form A has characteristic peaks at diffraction angles 2θ of about 4.41±0.2°, 5.34±0.2°, 8.90±0.2°, 9.16±0.2°, 10.69±0.2°, 11.07°±0.2°, 13.16±0.2°, 15.63±0.2°, 18.49±0.2°, 19.25±0.2°, 21.28±0.2°, 24.60±0.2°, and 26.85±0.2°, in particular, the hydrochloric hydrochloride crystal form A has an XRPD pattern substantially as shown in FIG. 7, and the target used in the XRPD is a Cu target.

The differential scanning calorimetry (DSC) graph of the hydrochloride crystal form A has endothermic peaks with peak temperatures of about 94.6 °C, about 237.4 °C and about 266.9 °C, respectively, in particular, the hydrochloride crystal form A has a DSC graph substantially as shown in FIG. 8.

The thermogravimetric (TGA) graph of the hydrochloride crystal form A shows that the sample has a weight loss of about 3.5% when heated to about 100 °C, in particular, the hydrochloride crystal form A has a TGA graph substantially as shown in FIG. 8.

As detected by ¹H NMR, in the hydrochloride crystal form A, the molar ratio of hydrochloric acid to the free base is about 1:1.

The present invention also provides a method for preparing the hydrochloride crystal form A, comprising: reacting the free base of Compound I with hydrochloric acid in a molar ratio of about 1:1 in a solvent selected from ether solvents (such as tetrahydrofuran (THF)) and ester solvents (such as ethyl acetate (EtOA)).

The X-ray powder diffraction (XRPD) pattern of the hydrochloride crystal form B has characteristic peaks at diffraction angles 2θ of about 3.73±0.2°, 4.96±0.2°, 7.24±0.2°, 10.26±0.2°, and 15.47±0.2°, in particular, the hydrochloride crystal form B has an XRPD pattern substantially as shown in FIG. 7, and the target type used in the XRPD is a Cu target.

The differential scanning calorimetry (DSC) graph of the hydrochloride crystal form B has endothermic peaks with peak temperatures of about 109.9 °C, about 160.3 °C and about 266.9 °C, respectively, in particular, the hydrochloride crystal form B has a DSC graph substantially as shown in FIG. 9.

The thermogravimetric (TGA) graph of the hydrochloride crystal form B shows that the sample has a weight loss of about 9.1% when heated to about 130 °C, in particular, the hydrochloride crystal form B has a TGA graph substantially as shown in FIG 9.

As detected by ¹H NMR, in the hydrochloride crystal form B, the molar ratio of hydrochloric acid to the free base is about 3:1.

The present invention also provides a method for preparing the hydrochloride crystal form B, comprising: reacting the free base of Compound I with hydrochloric acid in a molar ratio of about 3:1 in a solvent selected from ketone solvents (such as acetone), ether solvents (such as tetrahydrofuran (THF)), ester solvents (such as ethyl acetate (EtOA)) and mixed solvents (such as halogenated alkanes and alcohol solvents, such as dichloromethane/MeOH).

In one embodiment, the salt of Compound I is a phosphate.

In one embodiment, the phosphate of Compound I exists in the form of crystal form A.

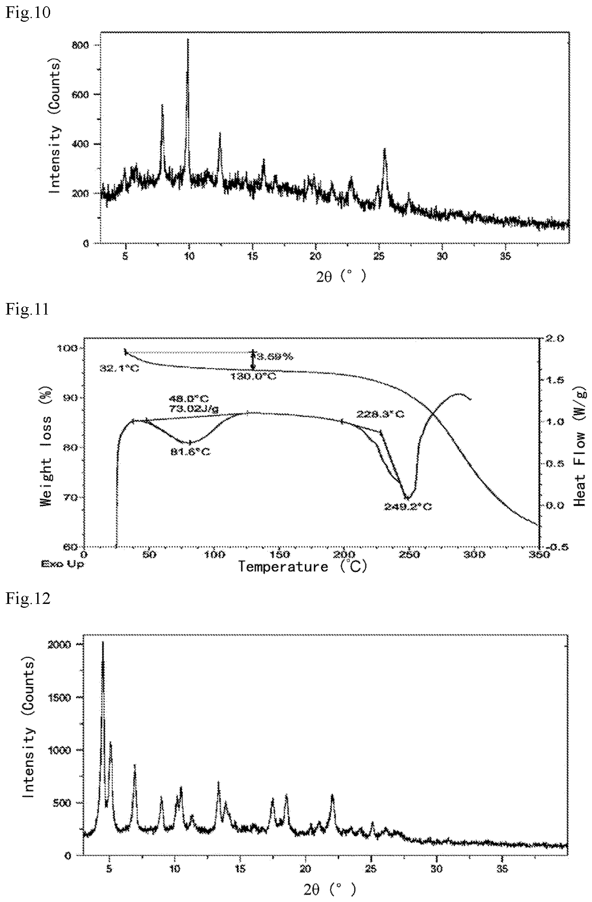

The X-ray powder diffraction (XRPD) pattern of the phosphate crystal form A has characteristic peaks at diffraction angles 2θ of about 5.63±0.2°, 7.86±0.2°, 9.88±0.2°, 12.43±0.2°, 15.86±0.2°, 19.64±0.2°, 21.26±0.2°, 22.72±0.2°, 25.42±0.2°, and 27.32±0.2°, in particular, the phosphate crystal form A has an XRPD pattern substantially as shown in FIG. 10, and the target type used in the XRPD is a Cu target.

The differential scanning calorimetry (DSC) graph of the phosphate crystal form A has endothermic peaks with onset temperatures of about 48.0 °C and about 228.3 °C, respectively, in particular, the phosphate crystal form A has a DSC graph substantially as shown in FIG.11.

The thermogravimetric (TGA) graph of the phosphate crystal form A shows that the sample has a weight loss of about 3.6% when heated to about 130 °C, in particular, the phosphate crystal form A has a TGA graph substantially as shown in FIG. 11.

As detected by ¹H NMR, in the phosphate crystal form A, the molar ratio of phosphoric acid to the free base is about 1:1.

The present invention also provides a method for preparing the phosphate crystal form A, comprising: reacting the free base of Compound I with phosphoric acid in a molar ratio of about 1:1 in a solvent selected from ketone solvents (such as acetone), ether solvents (such as tetrahydrofuran (THF)), ester solvents (such as ethyl acetate (EtOA)), and mixed solvents (such as halogenated alkanes and alcohol solvents, such as dichloromethane/MeOH).

In one embodiment, the salt of Compound I is a lactate.

In one embodiment, the lactate of Compound I exists in the form of crystal form A.

The X-ray powder diffraction (XRPD) pattern of the lactate crystal form A has characteristic peaks at diffraction angles 2θ of about 4.52±0.2°, 5.12±0.2°, 6.94±0.2°, 8.97±0.2°, 10.16±0.2°, 10.49°±0.2°, 11.30±0.2°, 13.35±0.2°, 13.86±0.2°, 17.51±0.2°, 18.52±0.2°, 21.02±0.2°, 21.97±0.2°, 25.10±0.2°, 26.05±0.2°, and 27.04±0.2°, in particular, the lactate crystal form A has an XRPD pattern substantially as shown in FIG. 12, and the target type used in the XRPD is a Cu target.

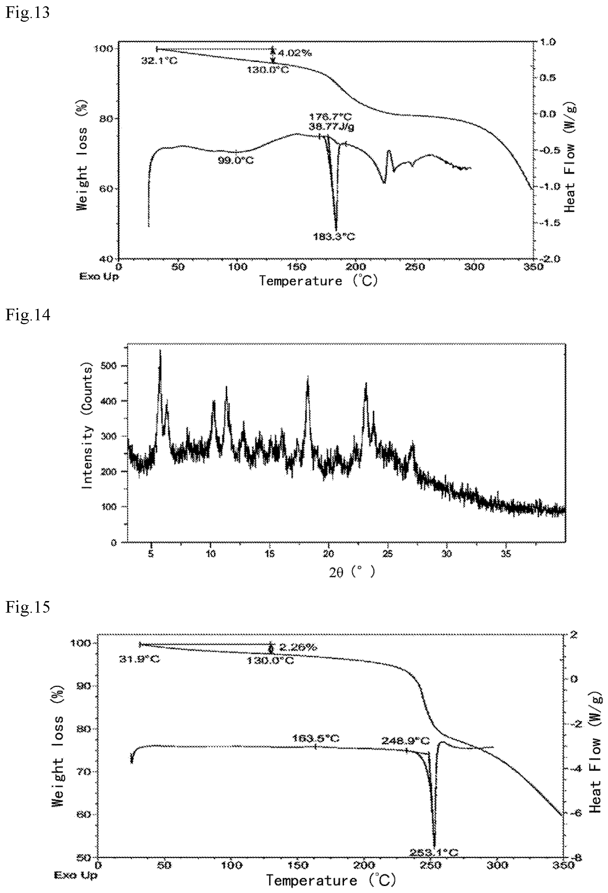

The differential scanning calorimetry (DSC) graph of the lactate crystal form A has endothermic peaks with peak temperatures of about 99.0 °C and about 183.3 °C, respectively, in particular, the lactate crystal form A has a DSC graph substantially as shown in FIG. 13.

The thermogravimetric (TGA) graph of the lactate crystal form A shows that the sample has a weight loss of about 4.0% when heated to about 130 °C, in particular, the lactate crystal form A has a TGA graph substantially as shown in FIG. 13.

As detected by ¹H NMR, in the lactate crystal form A, the molar ratio of lactic acid to the free base is about 1:1.

The present invention also provides a method for preparing the lactate crystal form A, comprising: reacting the free base of Compound I with lactic acid in a molar ratio of about 1: 1 in a solvent selected from ketone solvents (such as acetone), ether solvents (such as tetrahydrofuran (THF)), and ester solvents (such as ethyl acetate (EtOA)).

In one embodiment, the salt of Compound I is a fumarate.

In one embodiment, the fumarate of Compound I exists in the form of crystal form A.

The X-ray powder diffraction (XRPD) pattern of the fumarate crystal form A has characteristic peaks at diffraction angles 2θ of about 5.73±0.2°, 6.29±0.2°, 8.04±0.2°, 10.28±0.2°, 11.27±0.2°, 12.79±0.2°, 14.17±0.2°, 14.99±0.2°, 16.07±0.2°, 17.28±0.2°, 18.16±0.2°, 19.90±0.2°, 20.62±0.2°, 22.13±0.2°, 23.10±0.2°, 23.82±0.2°, 24.52±0.2°, and 27.03±0.2°, in particular, the fumarate crystal form A has an XRPD pattern substantially as shown in FIG. 14, and the target type used in the XRPD is a Cu target.

The differential scanning calorimetry (DSC) graph of the fumarate crystal form A has an exothermic peak with a peak temperature of about 163.5 °C, and an endothermic peak with an onset temperature of about 248.9 °C, in particular, the fumarate crystal form A has a DSC graph substantially as shown in FIG. 15.

The thermogravimetric (TGA) graph of the fumarate crystal form A shows that the sample has a weight loss of about 2.26% when heated to about 130 °C, in particular, the fumarate crystal form A has a TGA graph substantially as shown in FIG. 15.

As detected by ¹H NMR, in the fumarate crystal form A, the molar ratio of fumaric acid to the free base is about 1:1.

The present invention also provides a method for preparing the fumarate crystal form A, comprising: reacting the free base of Compound I with fumaric acid in a molar ratio of about 1:1 in a solvent selected from ether solvents (such as tetrahydrofuran (THF)), ester solvents (such as ethyl acetate (EtOA)), and mixed solvents (such as halogenated alkanes and alcohol solvents, such as dichloromethane/MeOH).

In one embodiment, the salt of Compound I is a succinate.

In one embodiment, the succinate of Compound I exists in the form of crystal form A.

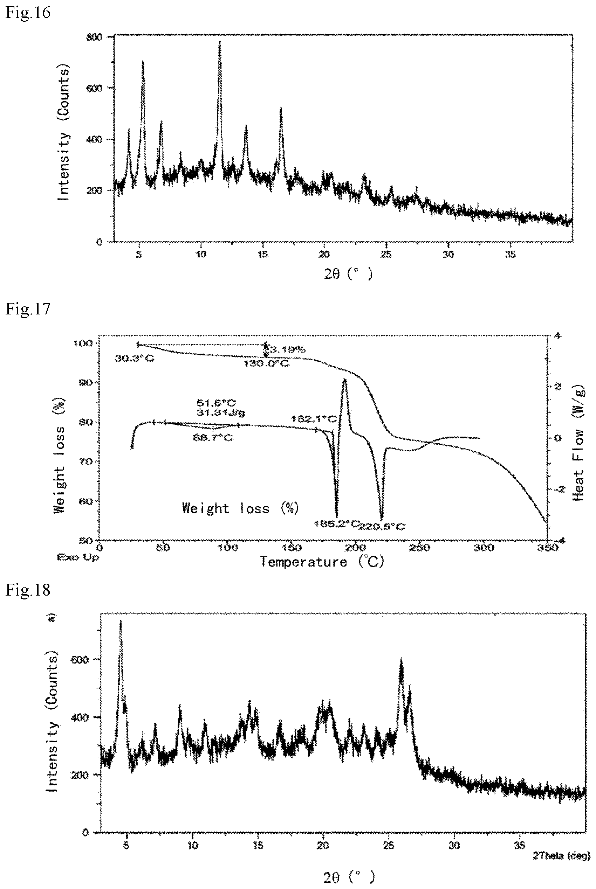

The X-ray powder diffraction (XRPD) pattern of the succinate crystal form A has characteristic peaks at diffraction angles 2θ of about 4.18±0.2°, 5.33±0.2°, 6.82±0.2°, 8.35±0.2°, 11.56±0.2°, 13.67°±0.2°, 16.44±0.2°, 17.74±0.2°, 20.47±0.2°, and 23.15±0.2°, in particular, the succinate crystal form A has an XRPD pattern substantially as shown in FIG. 16, and the target type used in the XRPD is a Cu target.

The differential scanning calorimetry (DSC) graph of the succinate crystal form A has endothermic peaks with onset temperatures of about 51.6 °C and about 182.1 °C, respectively, in particular, the succinate crystal form A has a DSC graph substantially as shown in FIG. 17.

The thermogravimetric (TGA) graph of the succinate crystal form A shows that the sample has a weight loss of about 3.2% when heated to about 130 °C, in particular, the succinate crystal form A has a TGA graph substantially as shown in FIG. 17.

As detected by ¹H NMR, in the succinate crystal form A, the molar ratio of succinic acid to the free base is about 1:1.

The present invention also provides a method for preparing the succinate crystal form A, comprising: reacting the free base of Compound I with succinic acid in a molar ratio of about 1:1 in a solvent selected from ketone solvents (such as acetone), ether solvent (such as tetrahydrofuran (THF)), and ester solvents (such as ethyl acetate (EtOA)).

In one embodiment, the salt of Compound I is a malate.

In one embodiment, the malate of Compound I exists in the form of crystal form A.

The X-ray powder diffraction (XRPD) pattern of the malate crystal form A has characteristic peaks at diffraction angles 2θ of about 4.49±0.2°, 6.10±0.2°, 7.16±0.2°, 9.00±0.2°, 10.99±0.2°, 14.87°±0.2°, 16.65±0.2°, 19.73±0.2°, 20.55±0.2°, 21.96±0.2°, 23.12±0.2°, 24.03±0.2°, 25.87±0.2°, and 26.58±0.2°, in particular, the malate crystal form A has an XRPD pattern substantially as shown in FIG. 18, and the target type used in the XRPD is a Cu target.

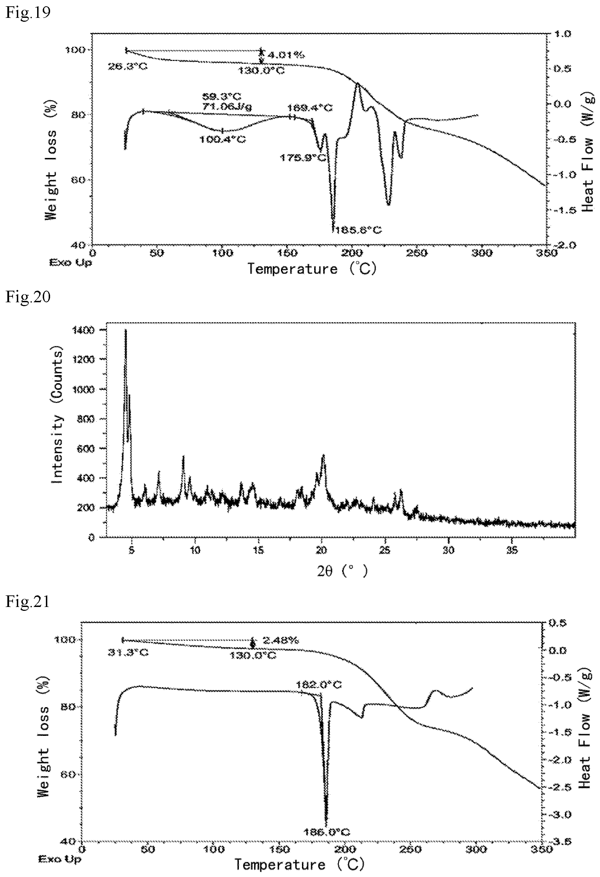

The differential scanning calorimetry (DSC) graph of the malate crystal form A has endothermic peaks with peak temperatures of about 100.4 °C, about 175.9 °C, and about 185.6 °C, respectively, in particular, the malate crystal form A has a DSC graph substantially as shown in FIG. 19.

The thermogravimetric (TGA) graph of the malate crystal form A shows that the sample has a weight loss of about 4.0% when heated to about 130 °C, in particular, the malate crystal form A has a TGA graph substantially as shown in FIG. 19.

As detected by ¹H NMR, in the malate crystal form A, the molar ratio of malic acid to the free base is about 1:1.

The present invention also provides a method for preparing the malate crystal form A, comprising: reacting the free base of Compound I with malic acid in a molar ratio of about 1:1 in a solvent selected from ketone solvents (such as acetone), ether solvents (such as tetrahydrofuran (THF)), and ester solvents (such as ethyl acetate (EtOA)).

In one embodiment, the salt of Compound I is an adipate.

In one embodiment, the adipate of Compound I exists in the form of crystal form A.

The X-ray powder diffraction (XRPD) pattern of the adipate crystal form A has characteristic peaks at diffraction angles 2θ of about 4.53±0.2°, 4.85±0.2°, 6.03±0.2°, 7.15±0.2°, 9.05±0.2°, 9.56±0.2°, 10.94±0.2°, 12.18±0.2°, 13.66±0.2°, 14.61±0.2°, 18.23±0.2°, 20.12±0.2°, 24.05±0.2°, 25.77±0.2°, 26.25±0.2°, and 27.50±0.2°, in particular, the adipate crystal form A has an XRPD pattern substantially as shown in FIG. 20, and the target type used in the XRPD is a Cu target.

The differential scanning calorimetry (DSC) graph of the adipate crystal form A has an endothermic peak with an onset temperature of about 182.0 °C, in particular, the adipate crystal form A has a DSC graph substantially as shown in FIG. 21.

The thermogravimetric (TGA) graph of the adipate crystal form A shows that the sample has a weight loss of about 2.5% when heated to about 130 °C, in particular, the adipate crystal form A has a TGA graph substantially as shown in FIG. 21.

As detected by ¹H NMR, in the adipate crystal form A, the molar ratio of adipic acid to the free base is about 1:1.

The present invention also provides a method for preparing the adipate crystal form A, comprising: reacting the free base of Compound I with adipic acid in a molar ratio of about 1:1 in a solvent selected from ketone solvents (such as acetone), ether solvents (such as tetrahydrofuran (THF)), and ester solvents (such as ethyl acetate (EtOA)).

In one embodiment, the salt of Compound I is a hippurate.

In one embodiment, the hippurate of Compound I exists in the form of crystal form A.

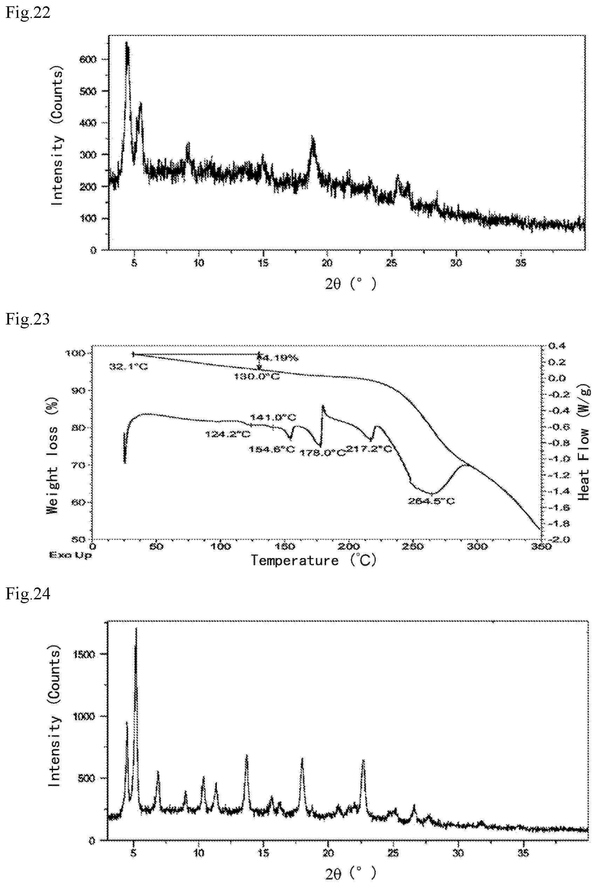

The X-ray powder diffraction (XRPD) pattern of the hippurate crystal form A has characteristic peaks at diffraction angles 2θ of about 4.45±0.2°, 5.50±0.2°, 9.17±0.2°, 18.79±0.2°, 23.20±0.2°, and 25.42±0.2°, in particular, the hippurate crystal form A has an XRPD pattern substantially as shown in FIG. 22, and the target type used in the XRPD is a Cu target.

The differential scanning calorimetry (DSC) graph of the hippurate crystal form A has endothermic peaks with peak temperatures of about 124.2 °C, about 141.0 °C, about 154.6 °C, about 178.0 °C, and about 217.2 °C, respectively, in particular, the hippurate crystal form A has a DSC graph substantially as shown in FIG. 23.

The thermogravimetric (TGA) graph of the hippurate crystal form A shows that the sample has a weight loss of about 4.2% when heated to about 130 °C, in particular, the hippurate crystal form A has a TGA graph substantially as shown in FIG. 23.

As detected by ¹H NMR, in the hippurate crystal form A, the molar ratio of hippuric acid to the free base is about 1:1.

The present invention also provides a method for preparing the hippurate crystal form A, comprising: reacting the free base of Compound I with hippuric acid in a molar ratio of about 1:1 in a solvent selected from ketone solvents (such as acetone), and ester solvents (such as ethyl acetate (EtOA)).

In one embodiment, the salt of Compound I is a glycolate.

In one embodiment, the glycolate of Compound I exists in the form of crystal form A.

The X-ray powder diffraction (XRPD) pattern of the glycolate crystal form A has characteristic peaks at diffraction angles 2θ of about 4.50±0.2°, 5.19±0.2°, 6.90±0.2°, 9.00±0.2°, 10.42±0.2°, 11.35°±0.2°, 13.74±0.2°, 15.60±0.2°, 16.24±0.2°, 17.97±0.2°, 20.77±0.2°, 22.68±0.2°, 25.12±0.2°, 26.61±0.2°, 27.69±0.2°, and 31.73±0.2°, in particular, the glycolate crystal form A has an XRPD pattern substantially as shown in FIG. 24, and the target used in the XRPD is a Cu target.

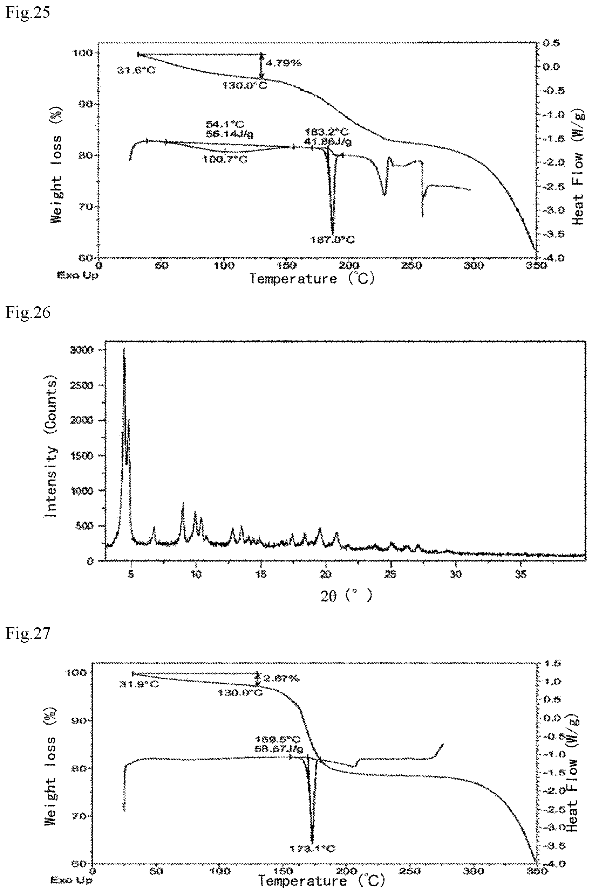

The differential scanning calorimetry (DSC) graph of the glycolate crystal form A has endothermic peaks with onset temperatures of about 54.1 °C and about 183.2 °C, respectively, in particular, the glycolate crystal form A has a DSC graph substantially as shown in FIG. 25.

The thermogravimetric (TGA) graph of the glycolate crystal form A shows that the sample has a weight loss of about 4.8% when heated to about 130 °C, in particular, the glycolate crystal form A has a TGA graph substantially as shown in FIG. 25.

As detected by ¹H NMR, in the glycolate crystal form A, the molar ratio of glycolic acid to the free base is about 1:1.

The present invention also provides a method for preparing the glycolate crystal form A, comprising: reacting the free base of Compound I with glycolic acid in a molar ratio of about 1:1 in a solvent selected from ketone solvents (such as acetone), ether solvents (such as tetrahydrofuran (THF)) and ester solvents (such as ethyl acetate (EtOA)).

In one embodiment, the salt of Compound I is a benzoate.

In one embodiment, the benzoate of Compound I exists in the form of crystal form A.

The X-ray powder diffraction (XRPD) pattern of the benzoate crystal form A has characteristic peaks at diffraction angles 2θ of about 4.47±0.2°, 4.80±0.2°, 6.74±0.2°, 8.96±0.2°, 9.94±0.2°, 10.40±0.2°, 12.78±0.2°, 13.50±0.2°, 14.88±0.2°, 17.41±0.2°, 18.32±0.2°, 19.54±0.2°, 20.84±0.2°, 23.58±0.2°, 25.01±0.2°, 26.31±0.2°, 27.11±0.2°, and 29.33±0.2°, in particular, the benzoate crystal form A has an XRPD pattern substantially as shown in FIG. 26, and the target type used in the XRPD is a Cu target.

The differential scanning calorimetry (DSC) graph of the benzoate crystal form A has an endothermic peak with an onset temperature of about 169.5 °C, in particular, the benzoate crystal form A has a DSC graph substantially as shown in FIG. 27.

The thermogravimetric (TGA) graph of the benzoate crystal form A shows that the sample has a weight loss of about 2.7% when heated to about 130 °C, in particular, the benzoate crystal form A has a TGA graph substantially as shown in FIG. 27.

As detected by ¹H NMR, in the benzoate crystal form A, the molar ratio of benzoic acid to the free base is about 1:1.

The present invention also provides a method for preparing the benzoate crystal form A, comprising: reacting the free base of Compound I with benzoic acid in a molar ratio of about 1:1 in a ketone solvent (such as acetone).

In one embodiment, the salt of Compound I is a nicotinate.

In one embodiment, the nicotinate of Compound I exists in the form of crystal form A.

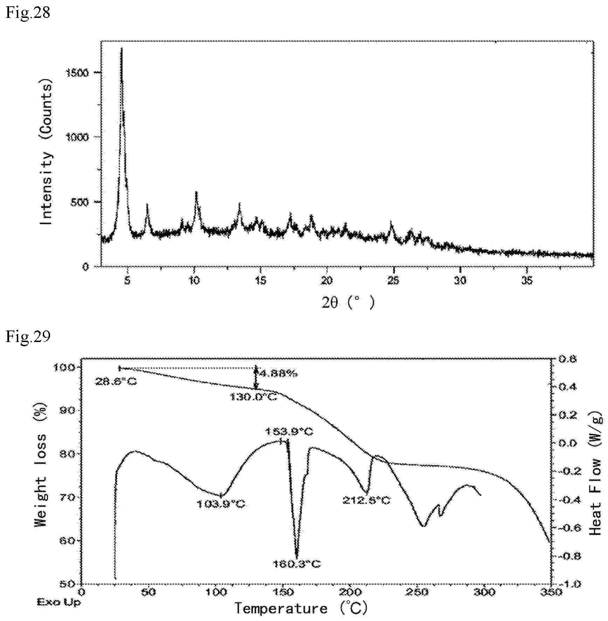

The X-ray powder diffraction (XRPD) pattern of the nicotinate crystal form A has characteristic peaks at diffraction angles 2θ of about 4.54±0.2°, 6.46±0.2°, 10.14±0.2°, 13.41±0.2°, 14.66±0.2°, 17.14°±0.2°, 18.83±0.2°, 21.36±0.2°, 24.83±0.2°, and 26.27±0.2°, in particular, the nicotinate crystal form A has an XRPD pattern substantially as shown in FIG. 28, and the target type used in the XRPD is a Cu target.

The differential scanning calorimetry (DSC) graph of the nicotinate crystal form A has endothermic peaks with peak temperatures of about 103.9 °C, about 160.3 °C and about 212.5 °C, respectively, in particular, the nicotinate crystal form A has a DSC graph substantially as shown in FIG. 29.

The thermogravimetric (TGA) graph of the nicotinate form A shows that the sample has a weight loss of about 4.9% when heated to about 130 °C, in particular, the nicotinate form A has a TGA graph substantially as shown in FIG. 29.

As detected by ¹H NMR, in the nicotinate crystal form A, the molar ratio of nicotinic acid to the free base is about 1:1.

The present invention also provides a method for preparing the nicotinate crystal form A, comprising: reacting the free base of Compound I with nicotinic acid in a molar ratio of about 1:1 in a solvent selected from ether solvents (such as tetrahydrofuran (THF)) and ester solvents (such as ethyl acetate (EtOA)).

In the methods for preparing the crystal forms of the present invention, seed crystals may also be used. Seed crystals can be used in the preparation methods of various crystal forms of the present application according to methods known in the art.

In another aspect, the present invention provides a pharmaceutical composition, comprising one or more salts of Compound I selected from the group consisting of maleate, hydrochloride, phosphate, lactate, fumarate, succinate, malate, adipate, hippurate, glycolate, benzoate and nicotinate.

In addition, the pharmaceutical composition may also include a pharmaceutically acceptable carrier. The pharmaceutically acceptable carrier may be selected variously according to administration routes and action characteristics, and can generally be a filler, a diluent, a binder, a wetting agent, a disintegrant, a lubricant, an emulsifier, a suspending agent, etc., which are conventional in the art.

The pharmaceutical composition can be administered by oral, injection (intravenous, intramuscular, subcutaneous and intracoronary), sublingual, buccal, rectal, urethral, vaginal, nasal, inhalation or topical route, and the preferred route is oral route.

The pharmaceutical composition can be used to prevent or treat diseases associated with abnormal cell cycle regulation. The "diseases associated with abnormal cell cycle regulation" may be "diseases associated with abnormal cyclin-dependent kinases (preferably CDK4 and/or CDK6), in particular tumors, more particularly, malignant tumors (such as breast cancer, colon cancer, non-small cell carcinoma, brain astrocytoma, chronic myelogenous leukemia, pancreatic cancer, acute monocytic leukemia, liver cancer (including hepatocellular carcinoma, hepatic adenocarcinoma), gastric cancer, non-small cell lung cancer, malignant glioblastoma and prostate adenocarcinoma, advanced solid tumors (including but not limited to breast cancer, central nervous system primary tumors/metastatic tumors, etc.).

The pharmaceutical composition can be used as an inhibitor of a cyclin-dependent kinase (preferably CDK4 and/or CDK6).

The pharmaceutical composition can be used to suppress the proliferation of tumor cells. The tumor cells are preferably cancer cells, which are preferably breast cancer cells, colon cancer cells, non-small cell carcinoma cells, brain astrocytoma cells, chronic myelogenous leukemia cells, pancreatic cancer cells, acute monocytic leukemia cells, liver cancer cells (including hepatocellular carcinoma cells, liver adenocarcinoma cells), gastric cancer cells, non-small cell lung cancer cells, malignant glioblastoma cells and prostate adenocarcinoma cells; and the breast cancer cells are preferably one or more of MCF-7, T-47D and ZR-75-1 breast cancer cells.

In another aspect, the present invention provides use of the salt of Compound I for preparation of a medicament, wherein the salt is selected from the group consisting of maleate, hydrochloride, phosphate, lactate, fumarate, succinate, malate, adipate, hippurate, glycolate, benzoate and nicotinate, and the medicament is used for preventing or treating a disease associated with abnormal cell cycle regulation, in particular, the "disease associated with abnormal cell cycle regulation" may be a "disease associated with an abnormal cyclin-dependent kinase (preferably CDK4 and/or CDK6), in particular a tumor, more specifically, a malignant tumor (such as breast cancer, colon cancer, non-small cell carcinoma, brain astrocytoma, chronic myelogenous leukemia, pancreatic cancer, acute monocytic leukemia, liver cancer (including hepatocellular carcinoma, hepatic adenocarcinoma), gastric cancer, non-small cell lung cancer, malignant glioblastoma and prostate adenocarcinoma, an advanced solid tumor (including but not limited to breast cancer, central nervous system primary tumor/metastatic tumor, etc.).

In another aspect, the present invention provides use of the salt of Compound I for preparation of an inhibitor of an cyclin-dependent kinase (preferably CDK4 and/or CDK6), wherein the salt is selected from the group consisting of maleate, hydrochloride, phosphate, lactate, fumarate, succinate, malate, adipate, hippurate, glycolate, benzoate and nicotinate.

In another aspect, the present invention provides use of the salt of Compound I for preparation of a medicament for suppressing the proliferation of tumor cells, wherein the salt is selected from the group consisting of maleate, hydrochloride, phosphate, lactate, fumarate, succinate, malate, adipate, hippurate, glycolate, benzoate and nicotinate. The tumor cells are preferably cancer cells; and the cancer cells are preferably breast cancer cells; and the breast cancer cells are preferably one or more of MCF-7, T-47D and ZR-75-1 breast cancer cells.

In the pharmaceutical composition and uses, the salt can be one or more selected from the group consisting of maleate crystal form A, maleate crystal form B, maleate crystal form C, hydrochloride crystal form A, hydrochloride crystal form B, phosphate crystal form A, lactate crystal form A, fumarate crystal form A, succinate crystal form A, malate crystal form A, adipate crystal form A, hippurate crystal form A, glycolate crystal form A, benzoate crystal form A and nicotinate crystal form A of Compound I.

More specifically, the salt can be one or more selected from the group consisting of the maleate crystal form A, fumarate crystal form A, adipate crystal form A and benzoate crystal form A of Compound I.

More particularly, the salt may be the maleate crystal form A of Compound I.

More particularly, the salt may be the fumarate crystal form A of Compound I.

More particularly, the salt may be the adipate crystal form A of Compound I.

More particularly, the salt may be the benzoate crystal form A of Compound I.

It should be understood that within the scope of the present invention, the above technical features of the present invention and the technical features specifically described below (such as in the examples) can be combined with each other to form new or preferred technical solutions. Owing to space constraints, they are not specified herein.

### Description of drawings

Fig. 1 is an XRPD pattern of the maleate crystal form A of Compound I according to the present invention.
Fig. 2 is DSC and TGA graphs of the maleate crystal form A of Compound I according to the present invention.
Fig. 3 is an XRPD pattern of the maleate crystal form B of Compound I according to the present invention.
Fig. 4 is DSC and TGA graphs of the maleate crystal form B of Compound I according to the present invention.
Fig. 5 is an XRPD pattern of the maleate crystal form C of Compound I according to the present invention.
Fig. 6 is DSC and TGA graphs of the maleate crystal form C of Compound I according to the present invention.
Fig. 7 is an XRPD pattern of the hydrochloride crystal form A and crystal form B of Compound I according to the present invention.
Fig. 8 is DSC and TGA graphs of the hydrochloride crystal form A of Compound I according to the present invention.
Fig. 9 is DSC and TGA graphs of the hydrochloride crystal form B of Compound I according to the present invention.
Fig. 10 is an XRPD pattern of the phosphate crystal form A of Compound I according to the present invention.
Fig. 11 is DSC and TGA graphs of the phosphate crystal form A of Compound I according to the present invention.
Fig. 12 is an XRPD pattern of the lactate crystal form A of Compound I according to the present invention.
Fig. 13 is DSC and TGA graphs of the lactate crystal form A of Compound I according to the present invention.
Fig. 14 is an XRPD pattern of the fumarate crystal form A of Compound I according to the present invention.
Fig. 15 is DSC and TGA graphs of the fumarate crystal form A of Compound I according to the present invention.
Fig. 16 is an XRPD pattern of the succinate crystal form A of Compound I according to the present invention.
FIG. 17 is DSC and TGA graphs of the succinate crystal form A of Compound I according to the present invention.
Fig. 18 is an XRPD pattern of the malate crystal form A of Compound I according to the present invention.
Fig. 19 is DSC and TGA graphs of the malate crystal form A of Compound I according to the present invention.
FIG. 20 is an XRPD pattern of the adipate crystal form A of Compound I according to the present invention.
FIG. 21 is DSC and TGA graphs of the adipate crystal form A of Compound I according to the present invention.
FIG. 22 is an XRPD pattern of the hippurate crystal form A of Compound I according to the present invention.
Fig. 23 is DSC and TGA graphs of the hippurate crystal form A of Compound I according to the present invention.
Fig. 24 is an XRPD pattern of the glycolate crystal form A of Compound I according to the present invention.
FIG. 25 is DSC and TGA graphs of the glycolate crystal form A of Compound I according to the present invention.
Fig. 26 is an XRPD pattern of the benzoate crystal form A of Compound I according to the present invention.
Fig. 27 is DSC and TGA graphs of the benzoate crystal form A of Compound I according to the present invention.
Fig. 28 is an XRPD pattern of the nicotinate crystal form A of Compound I according to the present invention.
Fig. 29 is DSC and TGA graphs of the nicotinate crystal form A of Compound I according to the present invention.

### Detailed Embodiments

The present invention is further illustrated below by means of examples, but the present invention is not limited to the scope of the examples. The experimental methods in the following examples of which the specific conditions are not specified were carried out according to conventional methods and conditions, or according to the product instructions.

Unless otherwise specified, the equipment and testing methods involved in the examples of the present invention are as follows:
X-ray powder diffraction (XRPD): the XRPD pattern was collected on a PANalytacal Empyrean X-ray powder diffraction analyzer, and the XRPD parameters are shown in Table 1.

**Table 1**

| **Parameters** | **XRPD(reflection mode)** |
|---|---|
| X-ray | Cu, kα, Kα1 (Å): 1.540598; Kα2 (Å): 1.544426 Kα2/Kα1 intensity ratio: 0.50 |
| X-ray tube setting | 45 kV, 40 mA |
| Divergence slit | automatic |
| Monochromator | none |
| Scan Mode | continuous |
| Scan range(°2θ) | 3° to 40° |
| Scan step(°2θ) | 0.013 |
| Scan time(min.) | ∼7 |

Thermogravimetric analysis (TGA) and differential scanning calorimetry (DSC): TGA and DSC graphs were collected on TA Q500/5000 thermogravimetric analyzer and TAQ200/2000 differential scanning calorimeter respectively, and the test parameters are shown in Table 2.

**Table 2: DSC and TGA test parameters**

| **Parameters** | **TGA** | **DSC** |
|---|---|---|
| Method | linear temperature rise | linear temperature rise |
| Sample pan | aluminum pan, open | aluminum pan, capped |
| Temperature range | room temperature to set temperature | 25 °C to set temperature |
| Scan Rate (°C/min) | 10 | |
| Protective gas | nitrogen | |

High-performance liquid chromatography (HPLC): the high-performance liquid chromatography was collected on an Agilent 1100HPLC, and the specific HPLC purity test parameters are shown in Table 3 below.

**Table 3**

| | | |
|---|---|---|
| Column | InersilODS-3 (4.6 ×250mm, 5µm) | |
| Mobile phase | A: 0.05% trifluoroacetic acid in water B: 0.05% trifluoroacetic acid in acetonitrile | |
| Gradient | Time(min) | % B |
| | 0.0 | 10 |
| | 15.0 | 35 |
| | 20.0 | 90 |
| | 23.0 | 90 |
| | 23.1 | 10 |
| | 26.0 | 10 |
| Time | 26.0 min | |
| Post run time | 0.0 min | |
| Flow rate | 1.0ml/ min | |
| Injection volume | 5µl | |
| Detection wavelength | 254nm(Reference: 500nm) | |
| Column temperature | 35°C | |
| Sample chamber temperature | room temperature | |
| Diluent | | acetonitrile : water=1:1(v/v) |

Ion chromatography (IC): the negative ion content was measured using ion chromatography (IC) on a Thermo ICS1100 system, and the molar ratio was determined combined with HPLC data. The specific instrument parameters are listed in Table 4.

**Table 4**

| **Parameters** | **Setting value** |
|---|---|
| Column | IonPac AS18 Analytical Column (4 ×250mm) |
| Mobile phase | 25 mmol NaOH |
| Injection volume | 25µl |
| Flow rate | 1.0µl/min |
| Sample chamber temperature | 35 °C |
| Column temperature | 35 °C |
| Current | 80 mA |
| Run time | 10min (maleic acid, adipic acid), 14min(fumaric acid), 18min(benzoic acid) |

Dynamic moisture sorption (DVS): the dynamic moisture sorption (DVS) curve was collected on DVS Intrinsic of SMS (Surface Measurement Systems). The relative humidity at 25 °C was corrected with the deliquescence points of LiCl, Mg(NO₃)₂ and KCI. DVS test parameters are listed in Table 5.

**Table 5**

| **Parameters** | **Setting value** |
|---|---|
| Temperature | 25 °C |
| Sample amount | 10 to 20 mg |
| Protective gas and flow rate | N₂, 200 ml/min |
| dm/dt | 0.002%/min |
| Minimum dm/dt equilibration time | 10 min |
| Maximum equilibration time | 180 min |
| RH range | 0%RH-95%RH-0%RH |
| RH gradient | 10%(0%RH-90%RH, 90%RH-0%RH) 5%(90%RH-95%RH, 95%RH-90%RH) |

Solution NMR: the solution NMR spectrum was collected on a Bruker 400M NMR instrument, and DMSO-d₆ was used as a solvent.

The heating test was carried out as follows:
Under nitrogen protection, the sample was heated to the target temperature and then lowered to room temperature, and the sample was taken out, exposed to air, and collected to perform XRPD measurement.

The free base of Compound 1 was prepared according to the method disclosed in CN106928219A, and its XRPD results showed only a few diffraction peaks, and its HPLC purity was 95.2 area%. TGA results showed that the sample had a weight loss of 4.8% when heated to 200°C. DSC results showed that the sample had one endothermic signal and two exothermic signals before melting at 233.6°C (onset temperature). The heating test results showed that no change was observed in the XPRD results when the starting sample was heated to 80 °C; and the diffraction peak at 2θ of about 4.7° disappeared and the crystallinity increased when the sample was heated to 135 °C and 175 °C.

### Preparation Example 1

Crystal forms were prepared using the following methods:
Acetone (acetone), tetrahydrofuran (THF), ethyl acetate (EtOA) and dichloromethane (DCM)/methanol (MeOH) (10:1, v/v) were used as solvents, respectively.

About 20 mg of the free base of Compound I was weighed, and added with the respective acid according to the respective molar ratio in Table 6, and 0.3 to 0.5 ml of the solvent. The resultant was stirred at room temperature for 2 days, and centrifuged to separate the solid, which was dried at 50 °C for 2 hours for XRPD, TGA, DSC and HPLC purity characterization. Blank indicates no acid was added.

The preparation and screening results of crystal forms are shown in Table 6.

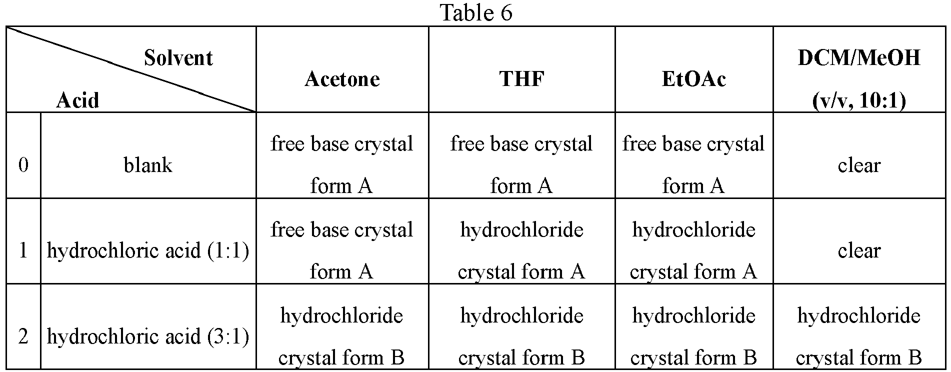

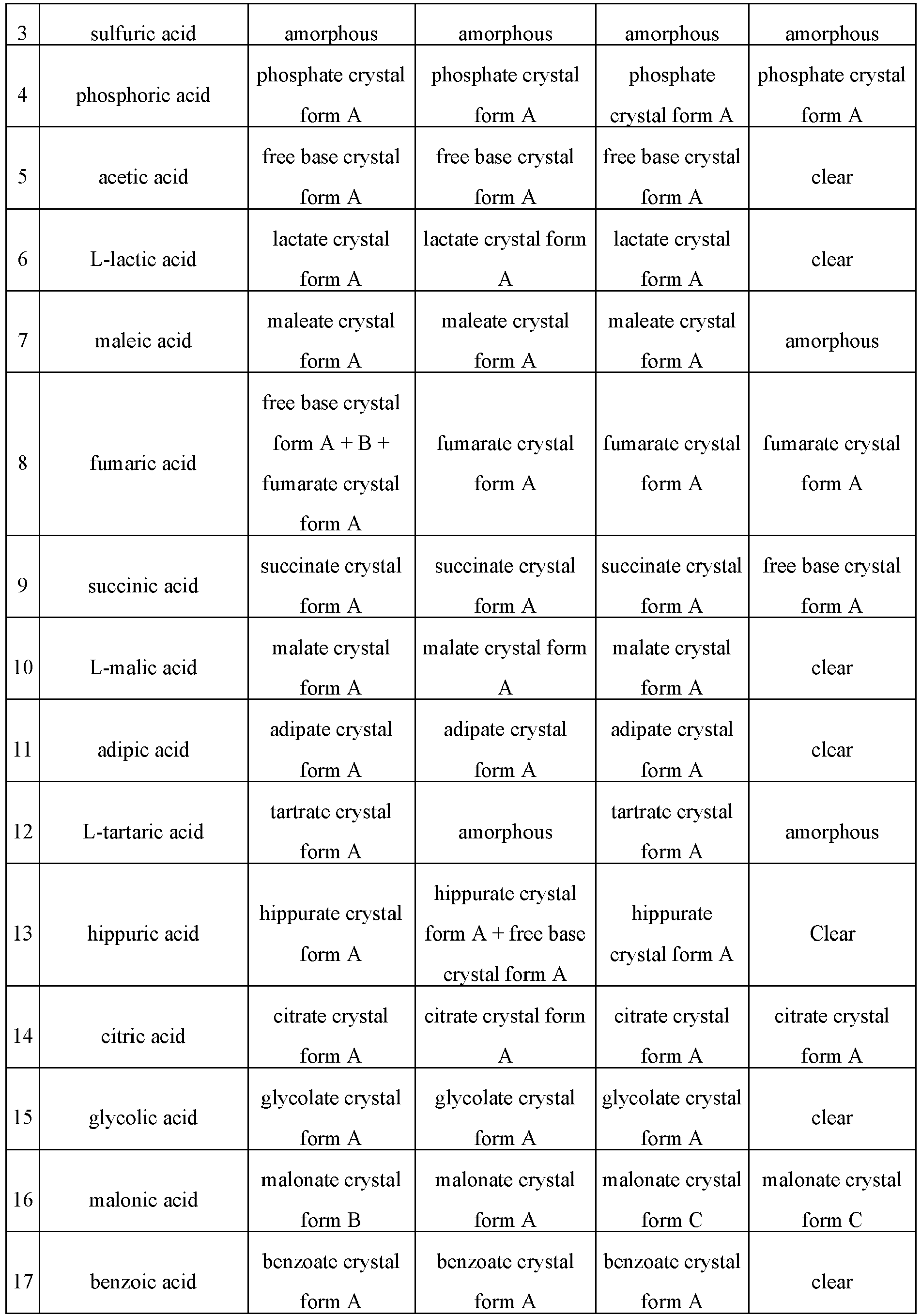

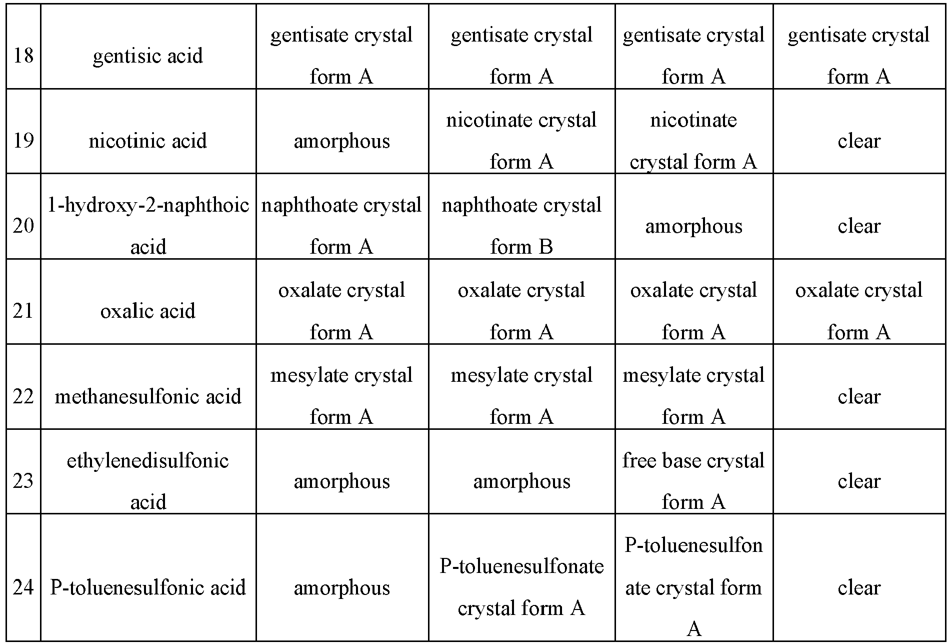

The XRPD results of the free base crystal form A showed a small number of diffraction peaks, and the HPLC purity was 98.2 area%. The TGA results showed that the sample had a weight loss of 1.2% when heated to 130°C. The DSC results showed that the sample had an endothermic peak before melting at 239.9°C (onset temperature). The DVS results showed that under the condition of 25 °C/80%RH, the water adsorption of the sample was 2.9%, and the XRPD results of the samples showed that the crystal form did not change before and after the DVS test.

After repeated preparation of crystal forms, it was found that the crystallinity of the samples in the system of tartaric acid, citric acid, gentisic acid and methanesulfonic acid was weak.

The safety level and crystallinity data of the acids used in the obtained samples of various crystal forms are summarized in Table 7 below.

**Table 7**

| **Crystal form** | **Safety level of acids** | **Crystalli nity** | **Weight loss %** | **DSC heat signal (peak, °C)** | |
|---|---|---|---|---|---|
| maleate crystal form A | I | high | 1.2 | 230.3 | |
| fumarate crystal form A | I | high | 2.2 | 253.6 | |
| adipate crystal form A | | I | high | 2.5 | 186.0 |
| benzoate crystal form A | | II | high | 2.7 | 173.1 |
| hydrochlori de | crystal form A | I | high | 3.5 | 94.6, 203.0, 237.4, 266.9 |
| | crystal form B | | low | 9.1 | 109.9, 160.3, 266.9 |
| phosphate crystal form A | | I | medium | 3.6 | 81.6, 249.2 |
| lactate crystal form A | | I | high | 4.0 | 99.0, 183.3 |
| succinate crystal form A | | I | high | 3.2 | 88.7, 185.2 |
| malate crystal form A | | I | medium | 4.0 | 100.4, 175.9, 185.6 |
| hippurate crystal form A | | I | low | 4.2 | 124.2, 141.0, 154.6, 178.0, 217.2, 264.5 |
| glycolate crystal form A | | I | high | 4.8 | 100.7, 187.0 |
| nicotinate crystal form A | | II | high | 4.9 | 103.9, 160.3, 212.5 |
| malonate | crystal form A | II | medium | - | - |
| | crystal form B | | medium | - | - |
| | crystal form C | | medium | - | - |
| naphthoate | crystal form A | II | medium | - | - |
| | crystal form B | | medium | - | - |
| oxalate crystal form A | | II | medium | - | - |
| p-toluenesulfonate crystal form A | | II | low | - | -- |

| | | | | | |
|---|---|---|---|---|---|
| Remarks: +: The data was not collected due to the small sample amount; --: The data was not collected considering the crystallinity of the sample and the safety level of the acid. | | | | | |

The XRPD characterization results of the maleate crystal form A are shown in FIG. 1, and the XRPD diffraction peak data are shown in Table 8. The TGA results are shown as the TGA graph in FIG. 2, indicating that the maleate crystal form A had a weight loss of 4.05% when heated to about 200 °C, and the DSC results are shown as the DSC graph in FIG. 2, indicating that the sample was melted at about 222.8 °C (onset temperature) and accompanied by decomposition. HPLC/IC results showed that the molar ratio of the sample was 1.03 (acid/base), and it is presumed that the maleate crystal form A is an anhydrous crystal form of monomaleate.

**Table 8: XRPD diffraction peak data of maleate crystal form A**

| **20(°)** | **Intensity [counts]** | **FWHM [°2θ]** | **d-spacing [Å]** | **Relative Intensity [%]** |
|---|---|---|---|---|
| **5.48** | **2511.55** | **0.1279** | **16.12** | **57.44** |
| 6.11 | 261.47 | 0.1023 | 14.46 | 5.98 |
| 7.84 | 258.71 | 0.1023 | 11.28 | 5.92 |
| 8.55 | 2178.79 | 0.1023 | 10.34 | 49.83 |
| 10.92 | 4372.65 | 0.1023 | 8.10 | 100.00 |
| 12.04 | 3171.95 | 0.1279 | 7.35 | 72.54 |
| 12.98 | 434.90 | 0.1023 | 6.82 | 9.95 |
| 13.81 | 415.32 | 0.1535 | 6.41 | 9.50 |
| 15.32 | 123.24 | 0.1535 | 5.78 | 2.82 |
| 16.40 | 571.44 | 0.1279 | 5.41 | 13.07 |
| 17.27 | 96.14 | 0.1535 | 5.14 | 2.20 |
| 18.70 | 145.22 | 0.1535 | 4.75 | 3.32 |
| 19.59 | 466.67 | 0.1279 | 4.53 | 10.67 |
| 21.55 | 105.55 | 0.1535 | 4.12 | 2.41 |
| 22.21 | 128.64 | 0.1535 | 4.00 | 2.94 |
| 22.80 | 183.91 | 0.1023 | 3.90 | 4.21 |
| 23.32 | 133.95 | 0.1535 | 3.81 | 3.06 |
| 24.57 | 244.72 | 0.1791 | 3.62 | 5.60 |
| 25.62 | 190.64 | 0.1023 | 3.48 | 4.36 |
| 26.07 | 139.46 | 0.1535 | 3.42 | 3.19 |
| 27.46 | 412.62 | 0.1023 | 3.25 | 9.44 |
| 28.23 | 175.06 | 0.2047 | 3.16 | 4.00 |
| 28.68 | 299.36 | 0.1279 | 3.11 | 6.85 |
| 33.08 | 61.65 | 0.3070 | 2.71 | 1.41 |
| 38.76 | 188.64 | 0.1279 | 2.32 | 4.31 |

The XRPD results of the fumarate crystal form A are shown in FIG. 14. The TGA results are shown as the TGA graph in FIG. 15, indicating that the fumarate crystal form A sample had a weight loss of 2.3% when heated to about 130°C. The DSC results are shown as the DSC graph in FIG. 15, indicating that there was an exothermic peak at about 163.5 °C (peak temperature) and an endothermic peak at about 248.9 °C (onset temperature). The results of the heating test showed that the crystal form of the sample remained unchanged when heated to about 170 °C, and the crystallinity increased significantly. It is speculated that the exothermic signal is due to the transformation of amorphous form to the crystal form. The HPLC/IC results showed that the molar ratio of the fumarate crystal form A was 1.07 (acid/base), and it is speculated that the fumarate crystal form A is an anhydrous form of monofumarate.

The XRPD characterization results of the adipate crystal form A are shown in FIG. 20. The TGA results are shown as the TGA graph in FIG. 21, indicating that the adipate crystal form A sample had a weight loss of about 2.5% when heated to about 130 °C. The DSC results are shown as the DSC graph in FIG. 21, indicating that there was an endothermic peak at about 182.0 °C (onset temperature), and it is speculated that the adipate crystal form A is an anhydrous crystal form. HPLC/IC results showed that the molar ratio of the adipate crystal form A was 0.88 (acid/base).

The XRPD results of the benzoate crystal form A are shown in FIG. 26. The TGA results are shown as the TGA graph in FIG. 27, indicating that benzoate form A had a weight loss of about 2.7% when heated to about 130 °C. The DSC results are shown as the DSC graph in FIG. 27, indicating that there was an endothermic peak at about 169.5 °C (onset temperature), and it is speculated that the benzoate crystal form A is an anhydrous crystal form. HPLC/IC results showed that the molar ratio of the benzoate form A was 0.81 (acid/base).

The XRPD results of the hydrochloride crystal form A are shown in FIG. 7. The TGA results are shown as the TGA graph in FIG. 8, indicating that the sample had a weight loss of about 3.5% when heated to about 100 °C, and the DSC results are shown as the DSC graph in FIG. 8, indicating that there were multiple endothermic and exothermic peaks on the DSC graph, in particular, there were endothermic peaks with peak temperatures of about 94.6 °C, about 237.4 °C, and about 266.9 °C, respectively, and exothermic peaks with peak temperatures of about 156.2 °C and 203.0 °C, respectively. In addition, the endothermic peaks with peak temperatures of about 237.4 °C and about 266.9 °C had the onset temperatures of about 233.7 °C and about 261.9 °C, respectively.

The XRPD results of the hydrochloride crystal form B sample are shown in FIG. 7. The TGA results are shown as the TGA graph in FIG. 9, indicating that the sample had a weight loss of about 9.1% when heated to about 130 °C, and the DSC results are shown as the DSC graph in FIG. 9, indicating that there were endothermic peaks at about 109.9 °C, about 160.3 °C and about 266.9 °C (peak temperature).

The XRPD results of the phosphate crystal form A are shown in FIG. 10. The TGA results are shown as the TGA graph in FIG. 11, indicating that the phosphate crystal form A sample had a weight loss of about 3.6% when heated to about 130 °C, and the DSC results are shown as the DSC graph in FIG. 11, indicating that there were endothermic peaks at about 48.0 °C and about 228.3 °C (onset temperature).

The XRPD results of the lactate crystal form A are shown in FIG. 12. The TGA results are shown as the TGA graph in FIG. 13, indicating that the lactate crystal form A sample had a weight loss of about 4.0% when heated to about 130 °C, and the DSC results are shown as the DSC graph in FIG. 13, indicating that there were endothermic peaks at about 99.0 °C and about 183.3 °C (peak temperature).

The XRPD results of the succinate crystal form A are shown in FIG. 16. The TGA results are shown as the TGA graph in FIG. 17, indicating that the succinate form A sample had a weight loss of about 3.2% when heated to about 130 °C, and the DSC results are shown as the DSC graph in FIG. 17, indicating that there were endothermic peaks at about 51.6 °C and about 182.1 °C (onset temperature). The heating test results showed that the crystal form of the sample did not change when heated to 100 °C.

The XRPD results of the malate crystal form A are shown in FIG. 18. The TGA results are shown as the TGA graph in FIG. 19, indicating that the malate crystal form A sample had a weight loss of about 4.0% when heated to about 130 °C, and the DSC results are shown as the DSC graph in FIG. 19, indicating that there were multiple endothermic peaks before decomposition.

The XRPD characterization results of the hippurate crystal form A are shown in FIG. 22. The TGA results are shown as the TGA graph in FIG. 23, indicating that the hippurate crystal form A sample had a weight loss of about 4.2% when heated to about 130 °C, and the DSC results are shown as the DSC graph in FIG. 23, indicating that there were multiple endothermic peaks before decomposition, in particular, endothermic peaks with peak temperatures of about 124.2 °C, about 141.0 °C, about 154.6 °C, about 178.0 °C and about 217.2 °C, respectively.

The XRPD results of the glycolate crystal form A are shown in FIG. 24. The TGA results are shown as the TGA graph in FIG. 25, indicating that the glycolate crystal form A sample had a weight loss of about 4.8% when heated to about 130 °C, and the DSC results are shown as the DSC graph in FIG. 25, indicating that there were endothermic peaks at about 54.1 °C and about 183.2 °C (onset temperature). The results of the heating test showed that the crystal form did not change when the sample was heated to 130 °C.

The XRPD results of the nicotinate crystal form A are shown in FIG. 28. The TGA results are shown as the TGA graph in FIG. 29, indicating that the nicotinate form A sample had a weight loss of about 4.9% when heated to about 130 °C, and the DSC results are shown as the DSC graph in FIG. 29, indicating that there were multiple endothermic peaks before decomposition, in particular, endothermic peaks with peak temperatures of about 103.9 °C, about 160.3 °C and about 212.5 °C, respectively.

After comprehensively considering the following points: 1) the safety level of the selected acids (level I > level II > level III); 2) sharp diffraction peaks in the XRPD pattern (at the same time, in order to ensure the representativeness of the samples and collected data, priority is given to the pattern with no obvious amorphous peak); 3) small and gentle weight loss in TGA; 4) single and sharp melting peak in DSC (anhydrous crystal form with low water or solvent absorption is preferred), it is found that maleate crystal form A, fumarate crystal form A, adipate crystal form A and benzoate crystal form A are preferred.

### Experimental example 1

For maleate crystal form A, fumarate crystal form A, adipate crystal form A and benzoate crystal form A, the solubility at 37 °C, solid state stability, and hygroscopicity of the samples were evaluated, respectively.

### Dynamic solubility at 37 °C

The dynamic solubility test was carried out in four vehicles: water, simulated gastric fluid (SGF), simulated fasting intestinal fluid (FaSSIF) and simulated fed intestinal fluid (FeSSIF), to evaluate solubility and disproportionation risk of the maleate crystal form A, fumarate crystal form A, adipate form A and benzoate crystal form A.

In the test, the free base crystal form A was used as a reference. About 32 mg of a solid and 4.0 ml of the vehicle were mixed in a 5 ml glass bottle, sealed and fixed on a rotating disk with a rotation speed of 25 rpm, mixed rotarily at 37 °C for 1 hour, 2 hours, 4 hours and 24 hours, and then the sample was taken. The turbid sample was separated by centrifugation, and the filtered supernatant was taken to measure the HPLC concentration and pH, and the crystal form of the solid was determined by XRPD. If the sample remained clear, the sample was taken at 24 hours to test concentration and pH value.

The HPLC was collected on an Agilent 1100HPLC, and the solubility test parameters are shown in Table 9 below.

**Table 9 Solubility Test Parameters**

| Column | InersilODS-3 (4.6 ×250mm, 5µm) | |
|---|---|---|
| Mobile phase | A: 0.05% trifluoroacetic acid in water | |
| | B: 0.05% trifluoroacetic acid in acetonitrile | |
| Gradient | Time (min) | % B |
| | 0.0 | 10 |
| | 6.0 | 90 |
| | 7.0 | 90 |
| | 7.1 | 10 |
| | 10.0 | 10 |
| Time | 10.0min | |
| Post run time | 0.0min | |
| Flow rate | 1.0ml/ min | |
| Injection volume | 5µl | |
| Detection wavelength | 254 nm(Reference: 500nm) | |
| Column temperature | 35°C | |
| Sample chamber temperature | room temperature | |
| Diluent | acetonitrile : water=1:1(v/v) | |

The dynamic solubility test results at 37 °C of crystal form A of the four salts and the free base crystal form A are summarized in Table 10.

**Table 10: Summary of Dynamic Solubility Test Results**

| Dynamic Solubility in Water | | | | | | | | | | | | |
|---|---|---|---|---|---|---|---|---|---|---|---|---|
| Sample | 1 hour | | | 2 hours | | | 4 hours | | | 24 hours | | |
| | S¹ | pH² | FC³ | S¹ | pH² | FC³ | S¹ | pH² | FC³ | S¹ | pH² | FC³ |
| free base | 0.0031 | 8.5 | unchang ed | 0.0007 | 8.5 | uncha nged | 0.0019 | 8.2 | unchan ged | 0.0025 | 8.4 | change d |
| maleate | 4.3 | 4.8 | NA | 4.5 | 4.7 | NA | 4.6 | 4.6 | NA | 4.7 | 4.8 | unchan ged |
| fumarate | -- | -- | -- | -- | -- | -- | -- | -- | -- | >6.0 | 4.1 | -- |
| adipate | -- | -- | -- | -- | -- | -- | -- | -- | -- | >5.7 | 4.9 | -- |
| benzoate | 4.8 | 6.9 | NA | 5.8 | 6.9 | NA | 5.0 | 6.7 | NA | 5.1 | 7.2 | NA |

| Dynamic Solubility in SGF | | | | | | | | | | | | |
|---|---|---|---|---|---|---|---|---|---|---|---|---|
| Sample | 1 hour | | | 2 hours | | | 4 hours | | | 24 hours | | |
| | S¹ | pH² | FC³ | S¹ | pH² | FC³ | S¹ | pH² | FC³ | S¹ | pH² | FC³ |
| free base | 6.1 | 6.0 | unchang ed | 7.0 | 6.2 | NA | 6.8 | 6.5 | NA | 7.6 | 6.4 | NA |
| maleate | -- | -- | -- | -- | -- | -- | -- | -- | -- | >5.5 | 3.3 | -- |
| fumarate | -- | -- | -- | -- | -- | -- | -- | -- | -- | >6.1 | 3.4 | -- |
| adipate | -- | -- | -- | -- | -- | -- | -- | -- | -- | >6.2 | 3.9 | -- |
| benzoate | -- | -- | -- | -- | -- | -- | -- | -- | -- | >6.2 | 3.9 | -- |

| Dynamic Solubility in FaSSIF | | | | | | | | | | | | |
|---|---|---|---|---|---|---|---|---|---|---|---|---|
| Sample | 1 hour | | | 2 hours | | | 4 hours | | | 24 hours | | |
| | S¹ | pH² | FC³ | S¹ | pH² | FC³ | S¹ | pH² | FC³ | S¹ | pH² | FC³ |
| free base | 3.9 | 7.4 | unchang ed | 5.2 | 7.6 | uncha nged | 5.4 | 7.6 | unchan ged | 4.7 | 7.6 | change d |
| maleate | 5.3 | 6.0 | NA | 4.8 | 6.0 | NA | 5.2 | 5.8 | NA | 5.6 | 6.0 | NA |
| fumarate | 5.7 | 5.4 | NA | 5.3 | 5.5 | NA | 5.4 | 5.4 | NA | 5.8 | 5.7 | NA |
| adipate | 5.3 | 5.8 | NA | 5.2 | 5.9 | NA | 5.4 | 5.8 | NA | 5.5 | 5.9 | NA |
| benzoate | 5.5 | 6.7 | NA | 5.5 | 6.8 | NA | 6.0 | 6.7 | NA | 6.0 | 6.8 | NA |

| Dynamic Solubility in FeSSIF | | | | | | | | | | | | |
|---|---|---|---|---|---|---|---|---|---|---|---|---|
| Sample | 1 hour | | | 2 hours | | | 4 hours | | | 24 hours | | |
| | S¹ | pH² | FC³ | S¹ | pH² | FC³ | S¹ | pH² | FC³ | S¹ | pH² | FC³ |
| free base | 2.4 | 5.5 | oil | 2.4 | 5.5 | oil | 2.4 | 5.5 | oil | 2.3 | 5.6 | oil |
| maleate | 1.9 | 5.1 | oil | 2.0 | 5.0 | oil | 1.8 | 5.0 | oil | 1.8 | 5.2 | oil |
| fumarate | 1.8 | 4.8 | oil | 1.5 | 4.9 | oil | 1.8 | 4.8 | oil | 1.2 | 5.0 | oil |
| adipate | 1.7 | 5.0 | oil | 1.6 | 4.9 | oil | 1.7 | 5.0 | oil | 1.2 | 5.2 | oil |
| benzoate | 1.9 | 5.2 | oil | 1.9 | 5.1 | oil | 1.9 | 5.1 | oil | 1.4 | 5.3 | oil |

| | | | | | | | | | | | | |
|---|---|---|---|---|---|---|---|---|---|---|---|---|
| Remarks: S¹: solubility, µg/ml, the measured data was the concentration of the free base, pH²: the measured pH value of each sampling point, FC³: the crystal form changes after XRPD detection. --: the sample was dissolved clear and the data was not collected. NA: the sample is too small to be detected. free base: free base crystal form A, maleate: maleate crystal form A, fumarate: fumarate crystal form A, adipate: adipate crystal form A, benzoate: benzoate crystal form A. | | | | | | | | | | | | |

The results showed that 1) in water, the solubilities of the crystal form A of the four salts were significantly improved (from a few micrograms to several mg/ml) compared with the free base crystal form A, and the maleate crystal form A did not change in crystal form after being suspended in water for 24 hours; 2) in other vehicles, the crystal form A of the four salts and the free base crystal form A had similar solubilities, all of which were several mg/ml. However, all samples were observed to form oil after being suspended in FeSSIF for 1 hour. Compared with the case in FaSSIF, the in vitro solubility test results showed that food effects may need to be considered for optimal absorption.

### One week solid state stability

Appropriate amounts of samples were weighed and placed in the open under the conditions of 25 °C/60%RH and 40 °C/75%RH. After one week, all samples were subjected to XRPD characterization and HPLC purity test to detect changes in crystal form and purity.

The one-week stability evaluation results of maleate crystal form A, fumarate crystal form A, adipate crystal form A, benzoate crystal form A and free base crystal form A are summarized in Table 11.

**Table 11**

| Crystal form | Initial purity (area%) | Test condition (one week) | HPLC purity | | Crystal form change |
|---|---|---|---|---|---|
| | | | area% | % compared with the initial | |
| free base crystal form A | 98.24 | 25°C/60%RH | 98.26 | 100.0 | unchanged |
| | | 40°C/75%RH | 98.16 | 99.9 | unchanged |
| maleate crystal form A | 98.10 | 25°C/60%RH | 98.17 | 100.1 | unchanged |
| | | 40°C/75%RH | 98.13 | 100.0 | unchanged |
| fumarate | 98.25 | 25°C/60%RH | 98.28 | 100.0 | unchanged |
| crystal form A | | 40°C/75%RH | 98.11 | 99.9 | unchanged |
| adipate crystal form A | 98.10 | 25°C/60%RH | 97.64 | 99.5 | unchanged |
| | | 40°C/75%RH | 89.08* | 90.8 | unchanged |
| benzoate crystal form A | 98.02 | 25°C/60%RH | 97.69 | 99.7 | unchanged |
| | | 40°C/75%RH | 95.30* | 97.2 | unchanged |

The results showed that the four crystal forms and the free base did not change in crystal form or HPLC purity at 25 °C/60%RH, indicating good physical and chemical stability; but the HPLC purities of the adipate crystal form A and benzoate crystal form A samples decreased after storage at 40 °C/75%RH.

### Hygroscopicity

In order to evaluate the stability of the samples with humidity at 25 °C, DVS was used to test maleate crystal form A, fumarate crystal form A, adipate crystal form A, and benzoate crystal form A. The samples were pre-dried at 0% RH to remove the adsorbed solvent or water before testing.

The following conclusions are drawn from the test results:
1) Under 80%RH, the water adsorption (wt%) of maleate crystal form A and fumarate crystal form A were 0.8% and 1.0%, respectively, thus they had slight hygroscopicity; while the water adsorption of adipate crystal form A and benzoate crystal form A were 9.1% and 7.7%, respectively, thus they had hygroscopicity. In addition, XRPD tests were performed before and after the DVS test. The results showed that the maleate crystal form A and the adipate crystal form A remained unchanged, and the fumarate crystal form A and the benzoate crystal form A became weaker in crystallinity.
2) Under the conditions of 80%RH to 95%RH, except for the maleate crystal form A which absorbed less water, the water absorption of the other three salt samples all increased rapidly.

Based on the results, the solubilities of maleate crystal form A, fumarate crystal form A, adipate crystal form A and benzoate crystal form A in water and biological vehicles are similar to or higher than those of the free base crystal form A; except for adipate and benzoate where the HPLC purity was observed to decrease, other crystal forms and the free base crystal form A showed better physical and chemical stability in the solid state stability evaluation; according to the water adsorption results, maleate crystal form A and fumarate crystal form A samples have slight hygroscopicity, while the rest of the salt samples and free base form A were observed to be hygroscopic.

Based on the evaluation results on dynamic solubility, solid stability and hygroscopicity, it is concluded that the maleate crystal form A has better properties in the following aspects:
1) Under the same operating conditions, the crystallinity of laboratory batch samples was relatively high, while fumarate crystal form A, adipate crystal form A and benzoate crystal form A had moderate crystallinity;
2) Compared with free base crystal form A, the solubility of maleate crystal form A in water at 37 °C was significantly improved (from a few micrograms to several mg/ml), and the crystal form did not change after 24 hours; the solubilities in other vehicles (simulated gastric juice SGF, simulated fasting state intestinal fluid FaSSIF and simulated fed state intestinal fluid FeSSIF) were similar;
3) The maleate crystal form A sample was placed under the conditions of 25 °C/60%RH and 40 °C/75%RH for one week without any change in crystal form or HPLC purity, showing good physical and chemical stability;
4) The results of the dynamic water adsorption (DVS) test at 25 °C showed that the maleate crystal form A sample had slight hygroscopicity, and the water adsorption under high humidity conditions (95%RH) was lower than those of other candidate salts and the free base.

### Preparation example 2

Crystalline forms were prepared using the following general method:
About 200 mg of the free base was weighed, and the corresponding acid and 5 ml of EtOAc were added in a molar ratio of 1:1 to obtain a suspension, which was stirred at room temperature for 30 minutes. About 2 mg of seed crystals were added and magnetically stirred overnight with temperature cycling (50-20 °C, 0.1 °C/min, 3 times; 500 rpm). The solid was separated by centrifugation, dried in vacuum at room temperature for 5 hours, and the crystal sample was collected for XRPD and DSC tests.

The maleate crystal form, the fumarate crystal form, the adipate crystal form and the benzoate crystal form were respectively prepared by the method. The XRPD and DSC test results showed that they were consistent with the above maleate crystal form A, fumarate crystal form A, adipate crystal form A and benzoate crystal form A references, respectively.

### Preparation example 3

### 3.1 Preparation of the maleate crystal form B

An anti-solvent was added dropwise to a clear solution of the maleate of Compound I in a positive solvent with stirring (~1000 rpm) until solids were precipitated. If there were still no solids precipitated after adding about 10 mL of the anti-solvent, then the addition was stopped. The precipitated solid was separated by centrifugation (~10000 rpm, 2 minutes) and tested by XRPD.

The results obtained are shown in Table 12, and maleate crystal forms A and B, amorphous and oil were obtained.

**Table 12**

| **Positive solvent (v/v)** | **Anti-solvent** | **Solid crystal form** |
|---|---|---|
| MeOH/DCM (1:1) | acetone | crystal form B |
| | CHCl₃ | amorphous |
| ACN/H₂O (1:1) | isopropanol(IPA) | crystal form A |
| | THF | crystal form A |
| MeOH/THF (2:1) | EtOAc | crystal form A |
| | toluene | crystal form B |
| DMSO | MIBK | amorphous |
| | toluene | oil |

In addition, the maleate crystal form A of Compound I was suspended in CHCl₃/n-hexane (1:1, v/v) and magnetically stirred (~1000 rpm) at 50 °C for about 4 days, after centrifugation (~ 10,000 rpm, 3 min), the solid was collected for XRPD test. As a result, the maleate crystal form B was also obtained.

The XRPD results of the maleate crystal form B are shown in FIG. 3. The TGA characterization results are shown as the TGA graph in FIG. 4, showing that the sample had a weight loss of about 6.4% when heated to about 200 °C (the weight loss of about 19.1% between 200 and 290 °C was presumed to be caused by deacidification, and the theoretical weight loss of deacidification was about 18.7%). The DSC characterization results are shown as the DSC graph in FIG. 4, showing two weak endothermic peaks at about 74.6 °C and about 236.8 °C (peak temperature), and a sharp endothermic peak at about 225.5 °C (onset temperature). ¹H NMR results showed that the molar ratio of maleic acid to the free base was 1:1.

### 3.2 Preparation of maleate crystal form C

The maleate crystal form B transformed into the maleate crystal form C after purged with N₂ at 30 °C for 20 min.

The XRPD results of the maleate crystal form C are shown in FIG. 5. The TGA characterization results are shown as the TGA graph in FIG. 6, which shows that the sample had a weight loss of about 6.3% when heated to about 200 °C (the weight loss of about 17.5% between 200 and 290 °C was presumed to be caused by deacidification, and the theoretical weight loss of deacidification was about 18.7%). The DSC characterization results are shown as the DSC graph in FIG. 6, which shows an exothermic peak at about 142.8 °C (peak temperature) and a sharp endothermic peak at about 222.0 °C (onset temperature). ¹H NMR results showed that the molar ratio of maleic acid to the free base was 1:1. The VT-XRPD results showed that the maleate crystal form C did not change when it was heated to about 110 °C under N₂ protection. According to the results, it is speculated that the maleate crystal form C is an anhydrous form.

The maleate crystal form C transformed into the maleate crystal form A after heated to about 170 °C under N₂ protection.

According to the results, it is speculated that the maleate crystal form B is a hydrate, which is removed of the bound water by N₂ purging to be transformed into an anhydrous crystal form.

### 3.3 Slow volatilization preparation test

A vial containing a clear solution of the maleate salt of Compound I in a solvent listed in Table 13 below was sealed with a parafilm and punched with 5 small holes, and placed at room temperature for slow volatilization. After the solvent was completely evaporated, the obtained solid was collected and subjected to XRPD test.

The test results are shown in Table 13. Crystal form A and an amorphous form were obtained in the slow evaporation test.

**Table 13**

| **Solvent (v/v)** | **Solid crystal form** |
|---|---|
| MeOH/DCM (1:1) | crystal form A |
| acetonitrile(CAN)/H₂O (1:1) | amorphous |
| MeOH/THF (2:1) | crystal form A |

### 3.4 Slow cooling preparation test

A clear solution of the maleate salt of Compound I in a solvent listed in the following table 14 was placed in a biochemical incubator, and cooled from 50 °C to 5 °C at a cooling rate of 0.1 °C/min, and the solution without precipitated solid was transferred to room temperature for volatilization, and the resulting solid was collected for XRPD testing.

The test results are shown in Table 14. An amorphous form was obtained in the slowly cooling test.

**Table 14**

| **Solvent (v/v)** | **Solid crystal form** |
|---|---|
| MeOH/DCM (1:1) | amorphous |
| MeOH/THF (2:1) | amorphous |
| dimethylacetamide(DMAc) | amorphous * |

| | |
|---|---|
| Remarks: *: obtained by vacuum drying at room temperature. | |

### Experimental example 2

### 1. Suspension competition assay

To further study the stability relationship between the anhydrous crystal forms A and C and the hydrate crystal form B of the maleate, a suspension competition test for various crystal forms was set up. The tests included the suspension competition test of the crystal forms A and C in acetone and EtOAc at room temperature and 50 °C, and the suspension competition test of the anhydrous crystal form A and the hydrate crystal form B in MeOH/H₂O (water activity (aw) = 0 to 1) at room temperature.

The specific steps were as follows: 1) saturated solutions of the maleate anhydrous crystal form A in different solvents were prepared at a specific temperature; 2) equal masses of the crystal forms A and B or C samples (about 4 mg each) were added to 0.5 mL of the saturated solutions to form suspensions, respectively; 3) the suspensions were suspended and stirred at room temperature and 50 °C for about 5 days (~800 rpm); 4) the remaining solid was separated for XRPD testing.

The results of the suspension competition tests are summarized in Table 15.

**Table 15**

| Initial crystal form | Solvent | Temperature | Final crystal form |
|---|---|---|---|
| crystal form A + crystal form C | acetone | room temperature | crystal form A |
| | | 50 °C | |
| | EtOAc | room temperature | |
| | | 50 °C | |
| crystal form A + crystal form B | MeOH (a_{w}=0) | room temperature | crystal form A |
| | MeOH/H₂O (a_{w}∼0.3) | | |
| | MeOH/H₂O (a_{w}∼0.6) | | |
| | H₂O (a_{w}=1) | | |

The results showed that, the physical mixture of the crystal forms A and C transformed into the crystal form A after being suspended and stirred in acetone and EtOAc at room temperature and 50 °C; at room temperature, the physical mixture of the crystal forms A and B transformed into the crystal form A after being suspended and stirred MeOH/H₂O (a_{w} = 0 to 1). Accordingly, it is speculated that the crystal form A is a thermodynamically stable crystal form at room temperature to 50 °C under room temperature water activity (a_{w}) = 0 to 1.

### 2. Equilibrium Solubility Test

The equilibrium solubility of the maleate crystal forms A, B and C in water was tested at room temperature.

In the test, a suspension of each crystal form was prepared, stirred magnetically at room temperature for 24 hours (rotational speed -800 rpm) and then centrifuged (10000 rpm, 5 min), and the supernatant was filtered (0.22 µm PTFE membrane) to measure the solubility and pH, and the solid was tested for XRPD.

The results of the equilibrium solubility test in water on the maleate crystal forms A, B, and C are summarized in Table 16.

**Table 16**

| **Initial crystal form** | **Solubility (mg/mL)*** | **pH** | **Crystal form transformation** |
|---|---|---|---|
| Crystal form A | 4.1 | 4.5 | no |
| Crystal form B | 4.5 | 4.4 | no |
| Crystal form C | 13.1 | 4.7 | NA |

| | | | |
|---|---|---|---|
| Remarks: *: Calculated by free base equivalent. NA: not tested due to little solids remaining. | | | |

### 3. Solid State Stability Test

This test is used to evaluate the solid state stability of the maleate crystal form A.

An appropriate amount of the maleate crystal form A was weighed and placed in closed state at 80 °C for 1 day, in open state at 25 °C/60%RH and 40 °C/75%RH for a week. The solid samples separated under different conditions were tested for purity by HPLC to evaluate chemical stability, and crystal forms were tested by XRPD to evaluate physical stability.

The results of the solid state stability evaluation of the maleate crystal form A are summarized in Table 17.

**Table 17**

| Crystal form | Initial purity (area%) | 80 °C/1 day % initial | Crystal form change | 25 °C/60%RH /1 week % initial | Crystal form change | 40 °C/75%RH /1 week % initial | Crystal form change |
|---|---|---|---|---|---|---|---|
| Crystal form A | 98.4 | 100.1 | no | 100.0 | no | 100.0 | no |

Under the three test conditions, no crystal transformation occurred and the HPLC purity did not decrease, indicating that the maleate crystal form A has good physical and chemical stability under the selected test conditions.

### 4. Stability in solvents

This test is used to evaluate the stability of the maleate salt of Compound I in solvents.

Suspension stirring tests were performed in dichloromethane (DCM), methanol (MeOH), ethyl acetate (EtOAc), dimethylformamide (DMF), tetrahydrofuran (THF), MTBE, 1,4-dioxane, respectively, to evaluated the stability of the maleate of Compound I in solvents.

The specific steps were as follows: ~15 mg of the maleate crystal form A of Compound I was weighed, and added to different solvents, and after suspending and stirring at room temperature for 6 h, it was transferred to 60 °C and stirred for 5 h, the solid was separated by centrifuge for XRPD testing, and the solution was used for HPLC purity analysis.

The results showed that the crystal form of the maleate crystal form A remained unchanged after suspension and stirring in DCM, EtOAc, THF, and MTBE, and the purity did not decrease significantly; after suspension and stirring in MeOH and DMF, the crystal form remained unchanged, and the purity slightly increased; and after suspension and stirring in 1,4-dioxane, the crystal form remained unchanged, but the purity decreased slightly.

The results of stability evaluation of the maleate crystal form A in solvents are summarized in Table 18 and Table 19.

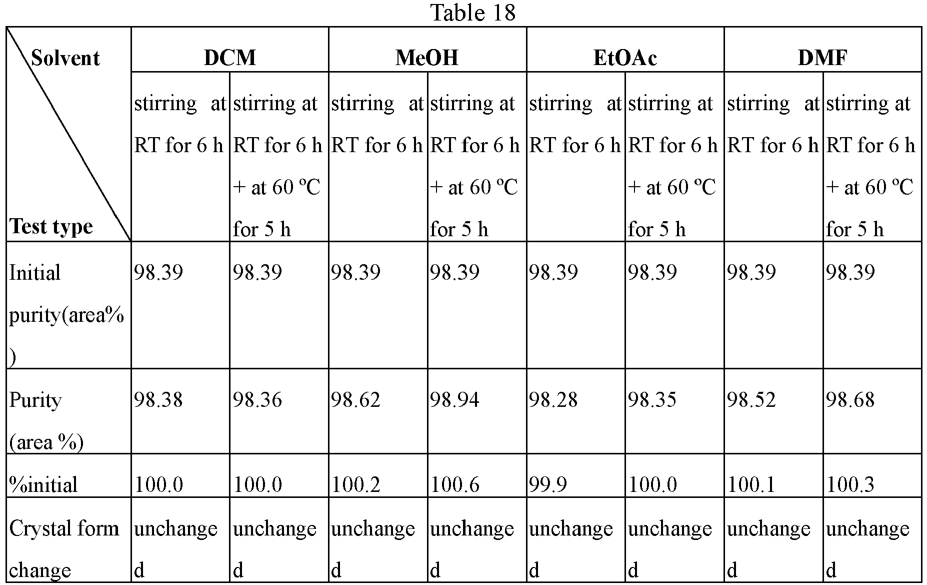

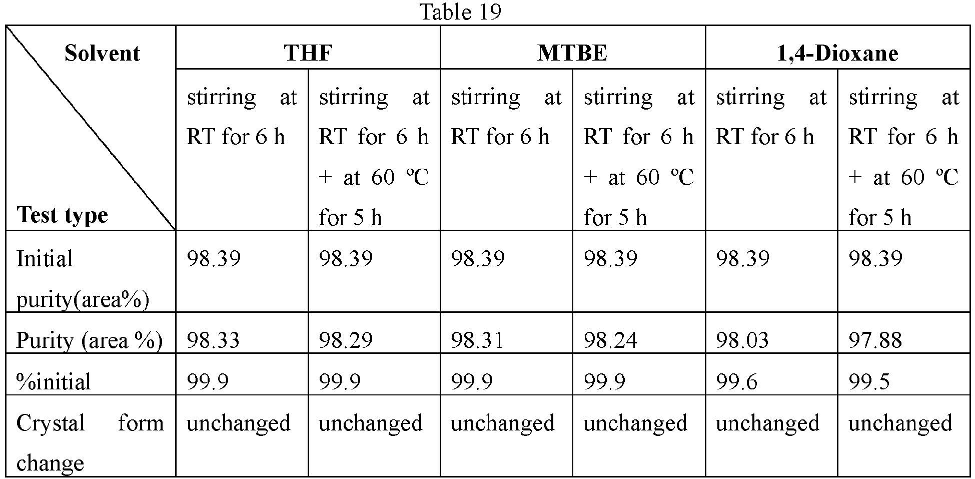

### Experimental example 3 Stability comparison between the free base crystal form A and the maleate crystal form A

The free base crystal form A and the maleate crystal form A were observed at high temperature (60 °C) under light irradiation (1.2*10⁶ Lux*hr) for a period of 5 days. The detection items were the related substances.

### Analytical method for the related substances of the free base

**Instrument:** High Performance Liquid Chromatography
**Chromatographic column:** Waters XBridge C18, 150*4.6mm, 3.5µm
**Mobile phase A:** 10mmol ammonium dihydrogen phosphate (containing 0.1% triethylamine): methanol (80:20)
**Mobile Phase B:** Methanol
**Gradient table:**

| Time(min) | Mobile Phase A(%) | Mobile Phase B(%) |
|---|---|---|
| 0 | 75 | 25 |
| 15 | 44 | 56 |
| 20 | 37 | 63 |
| 25 | 25 | 75 |
| 40 | 25 | 75 |
| 40.01 | 75 | 25 |
| 45 | 75 | 25 |

**Detection wavelength:** 230nm
**Flow rate:** 1.0 mL/min
**Injection volume:** 20µl
**Column temperature:** 45 °C
**Running time:** 45min
**Test sample solution:** about 25mg of the sample was placed in a 100ml volumetric flask, added with methanol to dissolve and dilute to the mark, and shaken thoroughly to be used as the test solution.

### Analysis method of the related substances of the maleate crystal form A

**Instrument:** High Performance Liquid Chromatography
**Chromatographic column:** Waters Xbridge C18, 4.6×150mm, 3.5µm
**Mobile phase A:** 0.01M ammonium dihydrogen phosphate aqueous solution (containing 0.1% triethylamine): methanol=80:20
**Mobile Phase B:** Methanol
**Gradient table:**

| Time(min) | Mobile phase A(%) | Mobile phase B(%) |
|---|---|---|
| 0 | 75 | 25 |
| 15 | 44 | 56 |
| 20 | 37 | 63 |
| 25 | 25 | 75 |
| 40 | 25 | 75 |
| 40.01 | 75 | 25 |
| 45 | 75 | 25 |

**Detection wavelength:** 230nm
**Flow rate:** 1.0ml/min
**Injection volume:** 20µl
**Column temperature:** 45 °C
**Running time:** 45min
**Diluent:** 0.01M aqueous ammonium dihydrogen phosphate solution (containing 0.1% triethylamine): methanol=30:70
**The test solution:** about 30mg of the sample was placed in a 100ml volumetric flask, added with the diluent to dissolve and dilute to the mark, and shaken thoroughly to be used as the test solution.

The data obtained are as follows:

### Related substance data of the free base crystal form A/the maleate crystal form A at high temperature under light irradiation

| **Free base crystal form A** | | | **Maleate crystal form A** | | |
|---|---|---|---|---|---|
| influence factor | main peak area(%) | total impurities (%) | influence factor | main peak area(%) | total impurities (%) |
| free base, 0 day | 99.67 | 0.33 | Maleate, 0 day | 99.68 | 0.31 |
| free base, 60°C, 5 days | 96.68 | 3.31 | Maleate, 60°C, 5 days | 98.83 | 1.17 |
| free base, irradiation for 5 days | 81.86 | 18.15 | Maleate, irradiation for 5 days | 95.53 | 4.47 |

It can be seen from the table that the free base showed instability in the test at high temperature (60 °C) under light irradiation (1.2*10⁶ Lux·hr) for 5 days, while the maleate crystal form was more stable than the free base form under the same conditions.

## Claims

1. A salt of Compound I as shown below, wherein the salt is selected from the group consisting of maleate, hydrochloride, phosphate, lactate, fumarate, succinate, malate, adipate, hippurate, glycolate, benzoate and nicotinate,

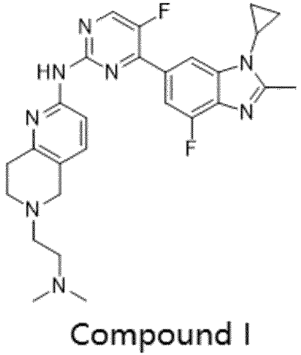

2. The salt of Compound I according to claim 1, wherein the salt is one or more selected from the group consisting of maleate crystal form A, maleate crystal form B, maleate crystal form C, hydrochloride crystal form A, hydrochloride crystal form B, phosphate crystal form A, lactate crystal form A, fumarate crystal form A, succinate crystal form A, malate crystal form A, adipate crystal form A, hippurate crystal form A, glycolate crystal form A, benzoate crystal form A and nicotinate crystal form A of Compound I,
particularly, the salt is one or more selected from the group consisting of maleate crystal form A, fumarate crystal form A, adipate crystal form A and benzoate crystal form A of Compound I,
more particularly, the salt is maleate crystal form A of Compound I,
more particularly, the salt is fumarate crystal form A of Compound I,
more particularly, the salt is adipate crystal form A of Compound I,
more particularly, the salt is benzoate crystal form A of Compound I.

3. The salt of Compound I according to claim 1 or 2, wherein,
the salt of Compound I is a maleate, in particular, the maleate of Compound I is one or more selected from the group consisting of crystal form A, crystal form B or crystal form C of the maleate;
the X-ray powder diffraction (XRPD) pattern of the maleate crystal form A has characteristic peaks at diffraction angles 2θ of 5.48±0.2°, 8.55±0.2°, 10.92±0.2°, 12.04±0.2°, 12.98±0.2°, 13.81±0.2°, 16.40±0.2°, 19.59±0.2°, and 27.46±0.2°, preferably, has further diffraction peaks at one or more positions of 6.11±0.2°, 7.84±0.2°, 15.32±0.2°, 18.70±0.2°, 21.55±0.2°, 22.21±0.2°, 22.80±0.2°, 23.32±0.2°, 24.57±0.2°, 25.62±0.2°, 26.07±0.2°, 28.23±0.2°, 28.68±0.2°, 33.08±0.2°, and 38.76±0.2°, preferably has characteristic peaks at diffraction angles 2θ of 5.48±0.2°, 6.11±0.2°, 7.84±0.2°, 8.55±0.2°, 10.92±0.2°, 12.04±0.2°, 12.98±0.2°, 13.81±0.2°, 15.32±0.2°, 16.40±0.2°, 18.70±0.2°, 19.59±0.2°, 21.55±0.2°, 22.21±0.2°, 22.80±0.2°, 23.32±0.2°, 24.57±0.2°, 25.62±0.2°, 26.07±0.2°, 27.46±0.2°, 28.23±0.2°, 28.68±0.2°, 33.08±0.2° and 38.76±0.2°, in particular, the maleate crystal form A has an XRPD pattern substantially as shown in FIG. 1, and the target type used in the XRPD is a Cu target; in particular, the differential scanning calorimetry (DSC) graph of the maleate crystal form A has an endothermic peak with an onset temperature of 222.8 °C and an endothermic peak with a peak temperature of 235.3 °C, in particular, the maleate crystal form A has a DSC graph substantially as shown in FIG. 2; in particular, the maleate crystal form A has a TGA graph substantially as shown in FIG. 2;
the X-ray powder diffraction (XRPD) pattern of the maleate crystal form B has characteristic peaks at diffraction angles 2θ of 5.75±0.2°, 9.72±0.2°, 14.91±0.2°, 15.76±0.2°, 17.43±0.2°, 18.09±0.2°, 22.20±0.2°, 23.23±0.2°, 25.17±0.2°, and 27.96±0.2°, in particular, the maleate crystal form B has an XRPD pattern substantially as shown in FIG. 3, and the target type used in the XRPD is a Cu target; in particular, the differential scanning calorimetry (DSC) graph of the maleate crystal form B has two weak endothermic peaks with peak temperatures of 74.6 °C and 236.8 °C, respectively, and a sharp endothermic peak with an onset temperature of 225.5 °C, in particular, the maleate crystal form B has a DSC graph substantially as shown in FIG. 4; in particular, the maleate crystal form B has a TGA graph substantially as shown in FIG. 4;
the X-ray powder diffraction (XRPD) pattern of the maleate crystal form C has characteristic peaks at diffraction angles 2θ of 5.51±0.2°, 6.31±0.2°, 8.91±0.2°, 9.62±0.2°, 10.59±0.2°, 11.05±0.2°, 12.17±0.2°, 14.99±0.2°, 15.73±0.2°, 16.65±0.2°, 21.40±0.2°, 22.94±0.2°, 24.71±0.2°, 26.72±0.2°, 28.22±0.2°, 32.36±0.2° and 38.52±0.2°, in particular, the maleate crystal form C has an XRPD pattern substantially as shown in FIG. 5, and the target used in the XRPD is a Cu target; in particular, the differential scanning calorimetry (DSC) graph of the maleate crystal form C has an exothermic peak with a peak temperature of 142.8 °C, and a sharp endothermic peak with an onset temperature of 222.0 °C, in particular, the maleate crystal form C has a DSC graph substantially as shown in FIG. 6; in particular, the maleate crystal form C has a TGA graph substantially as shown in FIG. 6;
or, the salt of Compound I is a hydrochloride, in particular, the hydrochloride of Compound I is one or more selected from the group consisting of hydrochloride crystal form A and hydrochloride crystal form B;
the X-ray powder diffraction (XRPD) pattern of the hydrochloride crystal form A has characteristic peaks at diffraction angles 2θ of 4.41±0.2°, 5.34±0.2°, 8.90±0.2°, 9.16±0.2°, 10.69±0.2°, 11.07°±0.2°, 13.16±0.2°, 15.63±0.2°, 18.49±0.2°, 19.25±0.2°, 21.28±0.2°, 24.60±0.2°, and 26.85±0.2°, in particular, the hydrochloric hydrochloride crystal form A has an XRPD pattern substantially as shown in FIG. 7, and the target used in the XRPD is a Cu target; in particular, the differential scanning calorimetry (DSC) graph of the hydrochloride crystal form A has endothermic peaks with peak temperatures of 94.6 °C, 237.4 °C and 266.9 °C, respectively, in particular, the hydrochloride crystal form A has a DSC graph substantially as shown in FIG. 8; in particular, the hydrochloride crystal form A has a TGA graph substantially as shown in FIG. 8;
the X-ray powder diffraction (XRPD) pattern of the hydrochloride crystal form B has characteristic peaks at diffraction angles 2θ of 3.73±0.2°, 4.96±0.2°, 7.24±0.2°, 10.26±0.2°, and 15.47±0.2°, in particular, the hydrochloride crystal form B has an XRPD pattern substantially as shown in FIG. 7, and the target type used in the XRPD is a Cu target; in particular, the differential scanning calorimetry (DSC) graph of the hydrochloride crystal form B has endothermic peaks with peak temperatures of 109.9 °C, 160.3 °C and 266.9 °C, respectively, in particular, the hydrochloride crystal form B has a DSC graph substantially as shown in FIG. 9; in particular, the hydrochloride crystal form B has a TGA graph substantially as shown in FIG 9;
or, the salt of Compound I is a phosphate, in particular, the phosphate of Compound I exists in the form of crystal form A, the X-ray powder diffraction (XRPD) pattern of the phosphate crystal form A has characteristic peaks at diffraction angles 2θ of 5.63±0.2°, 7.86±0.2°, 9.88±0.2°, 12.43±0.2°, 15.86±0.2°, 19.64±0.2°, 21.26±0.2°, 22.72±0.2°, 25.42±0.2°, and 27.32±0.2°, in particular, the phosphate crystal form A has an XRPD pattern substantially as shown in FIG. 10, and the target type used in the XRPD is a Cu target; in particular, the differential scanning calorimetry (DSC) graph of the phosphate crystal form A has endothermic peaks with onset temperatures of 48.0 °C and 228.3 °C, respectively, in particular, the phosphate crystal form A has a DSC graph substantially as shown in FIG. 11; in particular, the phosphate crystal form A has a TGA graph substantially as shown in FIG. 11;
or, the salt of Compound I is a lactate; in particular, the lactate of Compound I exists in the form of crystal form A, the X-ray powder diffraction (XRPD) pattern of the lactate crystal form A has characteristic peaks at diffraction angles 2θ of 4.52±0.2°, 5.12±0.2°, 6.94±0.2°, 8.97±0.2°, 10.16±0.2°, 10.49°±0.2°, 11.30±0.2°, 13.35±0.2°, 13.86±0.2°, 17.51±0.2°, 18.52±0.2°, 21.02±0.2°, 21.97±0.2°, 25.10±0.2°, 26.05±0.2°, and 27.04±0.2°, in particular, the lactate crystal form A has an XRPD pattern substantially as shown in FIG. 12, and the target type used in the XRPD is a Cu target; the differential scanning calorimetry (DSC) graph of the lactate crystal form A has endothermic peaks with peak temperatures of 99.0 °C and 183.3 °C, respectively, in particular, the lactate crystal form A has a DSC graph substantially as shown in FIG. 13; in particular, the lactate crystal form A has a TGA graph substantially as shown in FIG. 13;
or, the salt of Compound I is a fumarate, in particular, the fumarate of Compound I exists in the form of crystal form A, the X-ray powder diffraction (XRPD) pattern of the fumarate crystal form A has characteristic peaks at diffraction angles 2θ of 5.73±0.2°, 6.29±0.2°, 8.04±0.2°, 10.28±0.2°, 11.27±0.2°, 12.79±0.2°, 14.17±0.2°, 14.99±0.2°, 16.07±0.2°, 17.28±0.2°, 18.16±0.2°, 19.90±0.2°, 20.62±0.2°, 22.13±0.2°, 23.10±0.2°, 23.82±0.2°, 24.52±0.2°, and 27.03±0.2°, in particular, the fumarate crystal form A has an XRPD pattern substantially as shown in FIG. 14, and the target type used in the XRPD is a Cu target; in particular, the differential scanning calorimetry (DSC) graph of the fumarate crystal form A has an exothermic peak with a peak temperature of 163.5 °C, and an endothermic peak with an onset temperature of 248.9 °C, in particular, the fumarate crystal form A has a DSC graph substantially as shown in FIG. 15; in particular, the fumarate crystal form A has a TGA graph substantially as shown in FIG. 15;
or, the salt of Compound I is a succinate, in particular, the succinate of Compound I exists in the form of crystal form A, the X-ray powder diffraction (XRPD) pattern of the succinate crystal form A has characteristic peaks at diffraction angles 2θ of 4.18±0.2°, 5.33±0.2°, 6.82±0.2°, 8.35±0.2°, 11.56±0.2°, 13.67°±0.2°, 16.44±0.2°, 17.74±0.2°, 20.47±0.2°, and 23.15±0.2°, in particular, the succinate crystal form A has an XRPD pattern substantially as shown in FIG. 16, and the target type used in the XRPD is a Cu target; the differential scanning calorimetry (DSC) graph of the succinate crystal form A has endothermic peaks with onset temperatures of 51.6 °C and 182.1 °C, respectively, in particular, the succinate crystal form A has a DSC graph substantially as shown in FIG. 17; in particular, the succinate crystal form A has a TGA graph substantially as shown in FIG. 17;
or, the salt of Compound I is a malate, in particular, the malate of Compound I exists in the form of crystal form A, the X-ray powder diffraction (XRPD) pattern of the malate crystal form A has characteristic peaks at diffraction angles 2θ of 4.49±0.2°, 6.10±0.2°, 7.16±0.2°, 9.00±0.2°, 10.99±0.2°, 14.87°±0.2°, 16.65±0.2°, 19.73±0.2°, 20.55±0.2°, 21.96±0.2°, 23.12±0.2°, 24.03±0.2°, 25.87±0.2°, and 26.58±0.2°, in particular, the malate crystal form A has an XRPD pattern substantially as shown in FIG. 18, and the target type used in the XRPD is a Cu target; in particular, the differential scanning calorimetry (DSC) graph of the malate crystal form A has endothermic peaks with peak temperatures of 100.4 °C, 175.9 °C, and 185.6 °C, respectively, in particular, the malate crystal form A has a DSC graph substantially as shown in FIG. 19; in particular, the malate crystal form A has a TGA graph substantially as shown in FIG. 19;
or, the salt of Compound I is an adipate, in particular, the adipate of Compound I exists in the form of crystal form A, the X-ray powder diffraction (XRPD) pattern of the adipate crystal form A has characteristic peaks at diffraction angles 2θ of 4.53±0.2°, 4.85±0.2°, 6.03±0.2°, 7.15±0.2°, 9.05±0.2°, 9.56±0.2°, 10.94±0.2°, 12.18±0.2°, 13.66±0.2°, 14.61±0.2°, 18.23±0.2°, 20.12±0.2°, 24.05±0.2°, 25.77±0.2°, 26.25±0.2°, and 27.50±0.2°, in particular, the adipate crystal form A has an XRPD pattern substantially as shown in FIG. 20, and the target type used in the XRPD is a Cu target; in particular, the differential scanning calorimetry (DSC) graph of the adipate crystal form A has an endothermic peak with an onset temperature of 182.0 °C, in particular, the adipate crystal form A has a DSC graph substantially as shown in FIG. 21; in particular, the adipate crystal form A has a TGA graph substantially as shown in FIG. 21;
or, the salt of Compound I is a hippurate, the hippurate of Compound I exists in the form of crystal form A, the X-ray powder diffraction (XRPD) pattern of the hippurate crystal form A has characteristic peaks at diffraction angles 2θ of 4.45±0.2°, 5.50±0.2°, 9.17±9.2°, 18.79±0.2°, 23.20±0.2°, and 25.42±0.2°, in particular, the hippurate crystal form A has an XRPD pattern substantially as shown in FIG. 22, and the target type used in the XRPD is a Cu target; in particular, the differential scanning calorimetry (DSC) graph of the hippurate crystal form A has endothermic peaks with peak temperatures of 124.2 °C, 141.0 °C, 154.6 °C, 178.0 °C, and 217.2 °C, respectively, in particular, the hippurate crystal form A has a DSC graph substantially as shown in FIG. 23; in particular, the hippurate crystal form A has a TGA graph substantially as shown in FIG. 23.
or, the salt of Compound I is a glycolate, in particular, the glycolate of Compound I exists in the form of crystal form A, the X-ray powder diffraction (XRPD) pattern of the glycolate crystal form A has characteristic peaks at diffraction angles 2θ of 4.50±0.2°, 5.19±0.2°, 6.90±0.2°, 9.00±0.2°, 10.42±0.2°, 11.35°±0.2°, 13.74±0.2°, 15.60±0.2°, 16.24±0.2°, 17.97±0.2°, 20.77±0.2°, 22.68±0.2°, 25.12±0.2°, 26.61±0.2°, 27.69±0.2°, and 31.73±0.2°, in particular, the glycolate crystal form A has an XRPD pattern substantially as shown in FIG. 24, and the target used in the XRPD is a Cu target; in particular, the differential scanning calorimetry (DSC) graph of the glycolate crystal form A has endothermic peaks with onset temperatures of 54.1 °C and 183.2 °C, respectively, in particular, the glycolate crystal form A has a DSC graph substantially as shown in FIG. 25; in particular, the glycolate crystal form A has a TGA graph substantially as shown in FIG. 25;
or, the salt of Compound I is a benzoate, in particular, the benzoate of Compound I exists in the form of crystal form A, the X-ray powder diffraction (XRPD) pattern of the benzoate crystal form A has characteristic peaks at diffraction angles 2θ of 4.47±0.2°, 4.80±0.2°, 6.74±0.2°, 8.96±0.2°, 9.94±0.2°, 10.40±0.2°, 12.78±0.2°, 13.50±0.2°, 14.88±0.2°, 17.41±0.2°, 18.32±0.2°, 19.54±0.2°, 20.84±0.2°, 23.58±0.2°, 25.01±0.2°, 26.31±0.2°, 27.11±0.2°, and 29.33±0.2°, in particular, the benzoate crystal form A has an XRPD pattern substantially as shown in FIG. 26, and the target type used in the XRPD is a Cu target; in particular, the differential scanning calorimetry (DSC) graph of the benzoate crystal form A has an endothermic peak with an onset temperature of 169.5 °C, in particular, the benzoate crystal form A has a DSC graph substantially as shown in FIG. 27; in particular, the benzoate crystal form A has a TGA graph substantially as shown in FIG. 27;
or, the salt of Compound I is a nicotinate, in particular, the nicotinate of Compound I exists in the form of crystal form A; the X-ray powder diffraction (XRPD) pattern of the nicotinate crystal form A has characteristic peaks at diffraction angles 2θ of 4.54±0.2°, 6.46±0.2°, 10.14±0.2°, 13.41±0.2°, 14.66±0.2°, 17.14°±0.2°, 18.83±0.2°, 21.36±0.2°, 24.83±0.2°, and 26.27±0.2°, in particular, the nicotinate crystal form A has an XRPD pattern substantially as shown in FIG. 28, and the target type used in the XRPD is a Cu target; in particular, the differential scanning calorimetry (DSC) graph of the nicotinate crystal form A has endothermic peaks with peak temperatures of 103.9 °C, 160.3 °C and 212.5 °C, respectively, in particular, the nicotinate crystal form A has a DSC graph substantially as shown in FIG. 29; in particular, the nicotinate form A has a TGA graph substantially as shown in FIG. 29.

4. A method for preparing the crystal forms of claim 2 or 3, wherein,
the method for preparing the maleate crystal form A is one of the following methods:
Method I:
(1) the maleate crystal form A is obtained by reacting the free base of Compound I with maleic acid in a solvent selected from ketone solvents (such as acetone), ether solvents (such as tetrahydrofuran), alcohol solvents (such as methanol) and ester solvents (such as ethyl acetate);
Method II:
(2) the maleate crystal form A is obtained by the transformation of maleate crystal form B or C;
the method for preparing the maleate crystal form B comprises: adding an anti-solvent (such as acetone and toluene) dropwise to a solution of maleate of Compound I in a mixed solvent of methanol /dichloromethane in a volume ratio of 1:1 for recrystallization, and placing the obtained solid under ambient humidity at room temperature;
the method for preparing the maleate crystal form C comprises: purging the maleate crystal form B with an inert gas (such as N₂);
the method for preparing the hydrochloride crystal form A comprises: reacting the free base of Compound I with hydrochloric acid in a molar ratio of 1:1 in a solvent selected from ether solvents (such as tetrahydrofuran) and ester solvents (such as ethyl acetate);
the method for preparing the hydrochloride crystal form B comprises: reacting the free base of Compound I with hydrochloric acid in a molar ratio of 3:1 in a solvent selected from ketone solvents (such as acetone), ether solvents (such as tetrahydrofuran), ester solvents (such as ethyl acetate) and mixed solvents (such as halogenated alkanes and alcohol solvents, such as dichloromethane/methanol);
the method for preparing the phosphate crystal form A comprises: reacting the free base of Compound I with phosphoric acid in a molar ratio of 1:1 in a solvent selected from ketone solvents (such as acetone), ether solvents (such as tetrahydrofuran), ester solvents (such as ethyl acetate), and mixed solvents (such as halogenated alkanes and alcohol solvents, such as dichloromethane/methanol);
the method for preparing the lactate crystal form A comprises: reacting the free base of Compound I with lactic acid in a molar ratio of 1:1 in a solvent selected from ketone solvents (such as acetone), ether solvents (such as tetrahydrofuran), and ester solvents (such as ethyl acetate);
the method for preparing the fumarate crystal form A comprises: reacting the free base of Compound I with fumaric acid in a molar ratio of 1:1 in a solvent selected from ether solvents (such as tetrahydrofuran), ester solvents (such as ethyl acetate), and mixed solvents (such as halogenated alkanes and alcohol solvents, such as dichloromethane/methanol);
the method for preparing the succinate crystal form A comprises: reacting the free base of Compound I with succinic acid in a molar ratio of 1:1 in a solvent selected from ketone solvents (such as acetone), ether solvent (such as tetrahydrofuran), and ester solvents (such as ethyl acetate);
the method for preparing the malate crystal form A comprises: reacting the free base of Compound I with malic acid in a molar ratio of 1:1 in a solvent selected from ketone solvents (such as acetone), ether solvents (such as tetrahydrofuran), and ester solvents (such as ethyl acetate);
the method for preparing the adipate crystal form A comprises: reacting the free base of Compound I with adipic acid in a molar ratio of 1:1 in a solvent selected from ketone solvents (such as acetone), ether solvents (such as tetrahydrofuran), and ester solvents (such as ethyl acetate);
the method for preparing the hippurate crystal form A comprises: reacting the free base of Compound I with hippuric acid in a molar ratio of 1:1 in a solvent selected from ketone solvents (such as acetone), and ester solvents (such as ethyl acetate);
the method for preparing the glycolate crystal form A comprises: reacting the free base of Compound I with glycolic acid in a molar ratio of 1:1 in a solvent selected from ketone solvents (such as acetone), ether solvents (such as tetrahydrofuran) and ester solvents (such as ethyl acetate);
the method for preparing the benzoate crystal form A comprises: reacting the free base of Compound I with benzoic acid in a molar ratio of 1:1 in a ketone solvent (such as acetone);
the method for preparing the nicotinate crystal form A comprises: reacting the free base of Compound I with nicotinic acid in a molar ratio of 1:1 in a solvent selected from ether solvents (such as tetrahydrofuran) and ester solvents (such as ethyl acetate).

5. A pharmaceutical composition comprising one or more selected from the salts of Compound I of any one of claims 1 to 3.

6. The pharmaceutical composition according to claim 5 for prevention or treatment of a disease associated with abnormal cell cycle regulation;
in particular, the "disease associated with abnormal cell cycle regulation" is a "disease associated with an abnormal cyclin-dependent kinase (preferably CDK4 and/or CDK6), in particular, a tumor, more particularly, a malignant tumor (such as breast cancer, colon cancer, non-small cell carcinoma, brain astrocytoma, chronic myelogenous leukemia, pancreatic cancer, acute monocytic leukemia, liver cancer (including hepatocellular carcinoma, hepatic adenocarcinoma), gastric cancer, non-small cell lung cancer, malignant glioblastoma and prostate adenocarcinoma, advanced solid tumor (including but not limited to breast cancer, central nervous system primary tumor/metastatic tumor);
in particular, the pharmaceutical composition is used as an inhibitor of a cyclin-dependent kinase (preferably CDK4 and/or CDK6);
in particular, the pharmaceutical composition is used to suppress the proliferation of tumor cells, the tumor cells are preferably cancer cells; the cancer cells are preferably breast cancer cells, colon cancer cells, non-small cell carcinoma cells, brain astrocytoma cells, chronic myelogenous leukemia cells, pancreatic cancer cells, acute monocytic leukemia cells, liver cancer cells (including hepatocellular carcinoma cells, liver adenocarcinoma cells), gastric cancer cells, non-small cell lung cancer cells, malignant glioblastoma cells and prostate adenocarcinoma cells; and the breast cancer cells are preferably one or more of MCF-7, T-47D and ZR-75-1 breast cancer cells.

7. Use of the salt of Compound I of any one of claims 1 to 3 for preparation of a medicament.

8. Use according to claim 7, wherein the medicament is used for preventing or treating a disease associated with abnormal cell cycle regulation; in particular, the "disease associated with abnormal the cell cycle regulation" is a "disease associated with an abnormal cyclin-dependent kinase (preferably CDK4 and/or CDK6), in particular, a tumor, more particularly, a malignant tumor (such as breast cancer, colon cancer, non-small cell carcinoma, brain astrocytoma, chronic myelogenous leukemia, pancreatic cancer, acute monocytic leukemia, liver cancer (including hepatocellular carcinoma, hepatic adenocarcinoma), gastric cancer, non-small cell lung cancer, malignant glioblastoma and prostate adenocarcinoma, advanced solid tumor (including but not limited to breast cancer, central nervous system primary tumor/metastatic tumor).

9. Use of the salt of Compound I of any one of claims 1 to 3 for preparation of an inhibitor of a cyclin-dependent kinase (preferably CDK4 and/or CDK6).

10. Use of the salt of Compound I of any one of claims 1 to 3 for preparation of a medicament for suppressing the proliferation of tumor cells, the tumor cells are preferably cancer cells; the cancer cells are preferably breast cancer cells, colon cancer cells, non-small cell carcinoma cells, brain astrocytoma cells, chronic myelogenous leukemia cells, pancreatic cancer cells, acute monocytic leukemia cells, liver cancer cells (including hepatocellular carcinoma cells, liver adenocarcinoma cells), gastric cancer cells, non-small cell lung cancer cells, malignant glioblastoma cells and prostate adenocarcinoma cells; the breast cancer cells are preferably one or more of MCF-7, T-47D and ZR-75-1 breast cancer cells.
